(19) Europäisches Patentamt
European Patent Office
Office européen des brevets

(11) EP 3 722 417 A1

(12) EUROPEAN PATENT APPLICATION
published in accordance with Art. 153(4) EPC

(43) Date of publication:
14.10.2020 Bulletin 2020/42

(51) Int Cl.:
C12N 5/09 (2010.01)    A61K 45/00 (2006.01)
A61P 35/00 (2006.01)    C12Q 1/02 (2006.01)
C12Q 1/6886 (2018.01)    G01N 33/15 (2006.01)
G01N 33/50 (2006.01)

(21) Application number: 18886936.6

(22) Date of filing: 06.12.2018

(86) International application number:
PCT/JP2018/044894

(87) International publication number:
WO 2019/111998 (13.06.2019 Gazette 2019/24)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 08.12.2017 JP 2017236374
17.10.2018 JP 2018195647

(71) Applicant: Kyo Diagnostics K.K.
Kyoto-shi, Kyoto 606-8501 (JP)

(72) Inventors:
• MIYOSHI, Hiroyuki
Kyoto-shi
Kyoto 606-8501 (JP)

• TAKETO, Makoto
Kyoto-shi
Kyoto 606-8501 (JP)
• YAMAMOTO, Takehito
Kyoto-shi
Kyoto 606-8501 (JP)
• SAKAI, Yoshiharu
Kyoto-shi
Kyoto 606-8501 (JP)

(74) Representative: dompatent von Kreisler Selting
Werner -
Partnerschaft von Patent- und Rechtsanwälten
mbB
Deichmannhaus am Dom
Bahnhofsvorplatz 1
50667 Köln (DE)

(54) CANCER SPHEROID PRODUCTION METHOD AND METHOD FOR SELECTING COLON CANCER PATIENTS

(57)    The disclosure includes a method of preparing a cancer spheroid, a method of selecting a colorectal cancer patient being sensitive to an FGFR inhibitor, and a pharmaceutical composition for treating colorectal cancer comprising an FGFR inhibitor.

EP 3 722 417 A1

**Description**

Technical field

The present application claims the benefit of the Japanese patent applications No. 2017-236374 and No. 2018-195647, which are incorporated herein by reference in their entirety.

The present disclosure includes a method of preparing a cancer spheroid. The present disclosure also includes a method of selecting a colorectal cancer patient for chemotherapy, and a therapeutic method and composition for treating colorectal cancer.

Background

For personalized medicine, patient-derived xenograft (PDX) models prepared by transplanting cancer tissues excised from cancer patients into immunodeficient animals have been studied. The PDX models, however, require repeated transplantation of tumor tissues in order to prepare a sufficient number of model animals for carrying out drug sensitivity tests, and thus take time to prepare. Instead of PDX, spheroids prepared from cells of patient tissues have been considered. However, spheroids have not been successfully prepared with sufficient efficiency for clinical application.

Regarding production of tissue-derived spheroids, it has been reported that spheroids are prepared by culturing human colonic epithelial cells in a conditioned medium of L-WRN fibroblasts that produce Wnt3a, R-spondin-3 and Noggin, which are essential for proliferation of tissue stem cells, and a ROCK inhibitor Y27632 and a TGF-β type I receptor inhibitor SB431542. It has also been reported that spheroids are prepared by culturing tumor cells of a colorectal cancer model mouse in a basic medium supplemented with Y27632 and SB431542.

Colorectal cancer is one of the leading causes of cancer death worldwide. For example, in the US, the 5-year survival rate for patients with stage IV colorectal cancer is only 13%. This poor prognosis is due to the difficulty in selecting an appropriate chemotherapy for individual patients. The current selection criteria for EGFR-targeted therapies in colorectal cancer are the absence of activating mutations in RAS pathway genes in tumor DNA. However, in clinical, 40-50% of selected patients do not respond to anti-EGFR therapies.

Genomic sequences and gene expression information have also been used to predict response to chemotherapy. However, it is still difficult to identify reliable biomarkers or gene expression information due to the genetic or epigenetic complexity of patients with colorectal cancer.

The fibroblast growth factor (FGF) family is one of the factors that directly promote the growth of epithelial cells. The FGF signal is considered to play an important role in cancer cell proliferation, migration, and survival, and FGF and its receptor family are expected as therapeutic targets leading to new drug development. Fibroblast growth factor receptor (FGFR) family proteins are receptor tyrosine kinases and the FGFR family is composed of four types of receptors, FGFR1 to 4. Homeostatic activation of the FGFR signaling pathway is thought to be deeply involved in the growth of some cancer cells. However, there is no reliable method to assess the sensitivity of colorectal cancer patients for adaptation to chemotherapy that inhibits the FGF signal.

Citation List

Non Patent Literature

Non Patent Literature 1: Kondo J, et al., Proc Natl Acad Sci U S A. 2011; 108: 6235-40
Non Patent Literature 2: van de Wetering M, et al., Cell. 2015; 161: 933-45
Non Patent Literature 3: Fujii M, et al., Cell Stem Cell. 2016; 18: 827-38
Non Patent Literature 4: Pauli C, et al., Cancer Discov. 2017; 7: 462-77
Non Patent Literature 5: Miyoshi H, et al., Science. 2012; 338: 108-13
Non Patent Literature 6: Miyoshi H, Stappenbeck TS. Nat Protoc. 2013; 8: 2471-82
Non Patent Literature 7: VanDussen KL, et al., Gut. 2015; 64: 911-20

Summary

Technical Problem

An object of the present disclosure is to provide an efficient method to prepare a cancer spheroid.

Another object of the present disclosure is to provide a method of selecting a colorectal cancer patient who is adaptive to an FGFR inhibitor. A further object of the present disclosure is to provide a therapeutic method and composition for treating colorectal cancer.

Solution to Problem

In an aspect, the present disclosure relates to a method of preparing a cancer spheroid, comprising culturing a cell population obtained from a cancer tissue of a patient in a medium containing

a ROCK inhibitor, a TGF-β type I receptor inhibitor, EGF and bFGF; and

an agent selected from an adenosine receptor agonist, a PPAR agonist, a calcium-activated potassium-channel (KCa) activator, an NMDA-type glutamate receptor agonist, a histamine H3 receptor agonist, and a retinoic acid receptor (RXR) β2 agonist.

In a further aspect, the present disclosure relates to a method of evaluating drug sensitivity of a patient, or a method of screening a therapeutic agent for a cancer, comprising

preparing a cancer spheroid by the method as described above, and

contacting the cancer spheroid thus obtained or a cancer spheroid derived therefrom with a candidate agent *in vitro*.

In a further aspect, the present disclosure relates to a method of preparing a patient-derived spheroid xenograft (PDSX) model, comprising

preparing a cancer spheroid by the method as described above, and

transplanting the cancer spheroid thus obtained or a cancer spheroid derived therefrom to a non-human animal.

In a further aspect, the present disclosure relates to a method of evaluating drug sensitivity of a patient, or a method of screening a therapeutic agent for a cancer, comprising

preparing a PDSX model by the method as described above, and administering a candidate agent to the PDSX model thus obtained.

In a further aspect, the present disclosure relates to a method of detecting microsatellite instability (MSI), comprising

preparing a cancer spheroid by the method as described above, and

determining MSI status or a mutation of a target gene of a mismatch repair gene in the cancer spheroid thus obtained or a cancer spheroid derived therefrom.

In a further aspect, the present disclosure relates to a method of selecting a colorectal cancer patient being sensitive to an FGFR inhibitor, comprising

treating a cancer spheroid or a patient-derived spheroid xenograft (PDSX) model derived from a colorectal cancer patient with an FGFR inhibitor, and

determining response to the FGFR inhibitor of the cancer spheroid or the PDSX model, and

selecting the colorectal cancer patient when the response to the FGFR inhibitor is positive.

In a further aspect, the present disclosure relates to a pharmaceutical composition for treating colorectal cancer comprising an FGFR inhibitor.

Effect of Invention

The present disclosure provides an efficient method for preparing a cancer spheroid, and enables an efficient drug sensitivity test for personalized medicine and an efficient drug screening for drug development, and also enables a highly sensitive MSI test.

The present disclosure also provides a method for selecting a colorectal cancer patient being sensitive to an FGFR inhibitor, and therapeutic method and composition for treating colorectal cancer using an FGFR inhibitor.

BRIEF DESCRIPTION OF THE DRAWINGS

Fig. 1 shows histological features of colorectal cancer tumor initiating cell (CRC-TIC) spheroids. Shown are pairs of H&E-stained specimens of primary tumors (left) and spheroids (right) derived from the same patients. There were three representative histotypes; well-differentiated types, with (top) and without (middle) mature goblet cells (arrowheads), and a poorly-differentiated type (bottom). Scale bars, 100 μm.

Fig. 2A shows quantitative presentation of bioluminescence from luciferase-expressing spheroids from days 1 to 4 after passages into wells of a 96-well cell culture plate. The gray level in each well represents direct photon counts from the spheroids embedded in the corresponding Matrigel drop. The total luminescence represents the cell number in each well.

Fig. 2B shows growth monitoring of luciferase-expressing spheroids. Photon counts from normal (HC3N raw; left) and cancer (HC5T raw; right) spheroids were scored daily during post-passage days 1-4. In Figs. 2B and 2C, the coefficient of variation (%CV) is shown for each data point.

Fig. 2C shows growth rates calibrated to the initial cell numbers based on the photon counts on day 1. Sixteen replicated wells were tested for each line.

Fig. 3A shows representative phase-contrast micrographs of four cancer spheroid lines cultured in the presence of EGF and/or bFGF for 8 days. Scale bars, 200 μm.

Fig. 3B shows GEI (growth effect index) of CRC-TIC spheroid lines carrying the wild-type *RAS/RAF* genes in the presence of EGF and/or bFGF. Shown are the plots and means of GEI in three independent experiments. In Figs. 3B-3D, data were analyzed using one-way ANOVA (P values are shown in the graphs) followed by Tukey's post-test. The statistical significance between the mean values are indicated by a, b, c, and d. The value differences between the different letters are statistically significant ($P < 0.05$), whereas those between the same are not.

Fig. 3C shows GEI of the spheroid lines carrying mutant *RAS/RAF* genes in the presence of EGF and/or bFGF. Shown are the plots and means of GEI in three independent experiments.

Fig. 3D shows GEI of normal colonic epithelial cell spheroids in the presence of EGF and/or bFGF. Shown are the plots and means of GEI in three independent spheroid lines.

Fig. 4A shows growth-promoting effects of the compounds identified in the screening. Shown are the data sets with mean values > 1.25 (i.e., > 25% increase in growth). Shown are the plots and means of GEI in three independent spheroid lines. Asterisks indicate significant differences from the solvent-only group ($P < 0.05$; analyzed using Student's t-test). NS, not significant.

Fig. 4B shows dose-dependent effects of three compounds on the growth of two CRC-TIC spheroid lines (HC18T and HC21T). Shown are the plots and means of GEI in three independent experiments. Asterisks indicate significant differences from the solvent-only group ($P < 0.05$; analyzed using one-way ANOVA followed by Tukey's post-test).

Fig. 4C shows representative phase-contrast micrographs of spheroids after NECA or forskolin treatment. A CRC-TIC spheroid (HC52T) was cultured with solvent only, 1 μM NECA, or 1 μM forskolin for five days. Note the increased sizes of NECA- and forskolin-treated spheroids caused by cell flattening. Scale bars, 100 μm.

Fig. 4D shows growth stimulation by three supplementary factors on slow-growing spheroids. Luciferase-expressing spheroids were cultured in the presence of the indicated factor(s) for 3 days. Shown are the plots and means of GEI in three independent experiments calculated from the same data sets in Fig. 9B. Data were analyzed using one-way ANOVA (P values are shown in the graphs) followed by Tukey's post-test. The mean values between the different letters are statistically different ($P < 0.05$).

Fig. 5 shows mutations in 21 oncogenes and tumor suppressor genes of 36 CRC-TIC spheroid lines. Shown are mutations that affect the amino acid sequences of the encoded proteins. Genes were classified into functional categories shown at the top. A gray rectangle in each cell represents a heterozygous (partially filled) or homozygous (full-filled) mutation. *, †, ‡, Spheroid lines labeled with the same symbols were derived from the same patients.

Fig. 6A shows growth rates of CRC-TIC lines carrying the wild-type *RAS/RAF* genes in the presence of EGF and/or bFGF. Each bar shows the mean growth rates of six replicates of three independent experiments. In Figs. 6A-6C, spheroids of CRC-TICs and normal colonic epithelium were treated with EGF and/or bFGF throughout post-passage days 1-4. Growth rates of the luciferase-expressing spheroids were calculated from their bioluminescence levels on post-passage days 1 and 4.

Fig. 6B shows growth rates of CRC-TIC lines carrying mutant *RAS/RAF* genes in the presence of EGF and/or bFGF. Each bar shows the mean growth rates of six replicates of three independent experiments.

Fig. 6C shows growth rates of normal colonic epithelial spheroids cultured in the eL-WRN medium in the presence of EGF and/or bFGF. Each bar shows the mean growth rates of six replicates of three independent spheroid lines.

Fig. 7A shows dose-dependent effects of SB431542 on the growth of spheroid lines. Luciferase-expressing cancer and normal spheroids were cultured in the cancer medium and the eL-WRN medium each containing EGF and bFGF, respectively, in the presence of SB431542 at the indicated concentrations. Tested were eleven CRC-TIC spheroid lines (circle) and four normal spheroid lines (square). Shown are the mean GEI of four replicates. Note that the effects on cancer spheroids were less than those on normal spheroids.

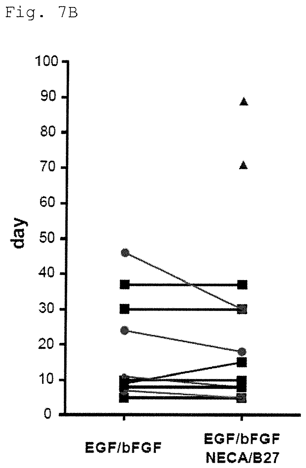

Fig. 7B shows growth-promoting effects by NECA and B27 supplement in culturing cells from colorectal cancer tissues. Epithelial cells separated from colorectal cancer tissues were cultured in the cancer medium containing EGF and bFGF in the presence or absence of NECA and B27 supplement. Shown is time required to obtain spheroids occupying four wells in a 12-well plate. Among the eleven spheroid lines, two lines were successfully established only in the presence of NECA and B27 (triangle), four lines proliferated more rapidly in the medium containing NECA and B27 (circle), and five lines were not affected by NECA and B27 (square).

Fig. 8A shows effects of 80 compounds on the growth of three independent CRC spheroid lines (GEI).

Fig. 8B shows the list of 80 compounds of Fig. 8A.

Fig. 9A shows dose-dependent effects of an adenosine receptor (AR) agonist CV1808 on the growth of two CRC spheroid lines (HC18T and HC21T). Shown are the mean GEI ± SEM (independent experiments, $n$ = 3). Asterisks indicate significant differences from the solvent-only (0 μM) group ($P$ < 0.05; analyzed using one-way ANOVA followed by Tukey's post-test).

Fig. 9B shows reproducibility of the dose-response effects of NECA in repeated experiments. The same data sets in Fig. 4B were used to perform curve-fitting analyses for the NECA treatment. Shown are fitted curves of the normalized data in three independent experiments.

Fig. 9C shows representative phase-contrast micrographs of CRC-TIC spheroids after treatments with cAMP-signaling activators. CRC spheroids (HC18T, HC21T, and HC52T) were cultured with solvent only (DMSO), 10 μM CV1808, 1 μM NECA, or 1 μM forskolin for five days. Scale bars, 200 μm.

Fig. 9D shows growth stimulation by three supplementary factors on slow-growing spheroid lines. Luciferase-expressing spheroids were cultured in the presence of the indicated factor(s) for 3 days. Shown are the means and plots of growth rates in three independent experiments. Data were analyzed using one-way ANOVA ($P$ values are shown in the graphs) followed by Tukey's post-test. The mean values between the different letters are statistically different ($P$ < 0.05). Note that growth rates in the control groups are ~1.0 (dotted lines), which implies their difficulty in growth.

Fig. 10A shows a schematic workflow for drug sensitivity tests in PDX and patient-derived spheroid xenograft (PDSX) models. The PDX and PDSX mice were prepared using the same tumor (left). PDX mice (top; P2-P3) and PDSX mice (bottom) were used for drug-dosing tests (right). Test mice were divided into three groups (control, FOLFOX, and cetuximab). Drug dosing started on day 1 and completed on day 22.

Fig. 10B shows results of drug-dosing tests with PDXs and PDSXs. Each data point shows the tumor volume on the corresponding day relative to that of day 1. n = 5-6 in each group. Error bars show SDs. *, $P$ < 0.05 and **; $P$ < 0.01. n.s., not significant in Tukey's multiple comparisons tests.

Fig. 11A shows results from two sets of drug-dosing tests with PDSX mice. n = 3-4 in each group.

Fig. 11B shows results of Sidak's multiple comparison test for the corresponding groups in the two sets of dosing tests with PDSXs. Error bars show SDs.

Fig. 11C shows results from two sets of drug-dosing tests with PDX mice (n = 2-4 in each group). Error bars show SDs. **, $P$ < 0.01 in Sidak's multiple comparisons tests.

Fig. 11D shows results of Sidak's multiple comparison test for the corresponding groups in the two sets of dosing tests with PDXs. Error bars show SDs.

Fig. 12A shows the serum CEA levels of a patient and chemotherapies given after resection of tumor (HC1T). The black diamond (a) indicates an interruption of chemotherapy due to an accidental a knee bone fracture. The timing of CT scans (shown in Fig. 12B and 12E) is indicated as "b-e" at the bottom.

Fig. 12B shows CT images of the patient before (b) and after (c) SOX (S-1 + oxaliplatin (Ox); similar to FOLFOX) treatment. White arrowheads point metastatic lesions in peritoneum. The best response to the SOX treatment in the patient was SD (stable disease; +11% with RECIST).

Fig. 12C shows results of drug-dosing tests with PDSXs derived from HC1T. The tumor growth in the FOLFOX mice was significantly decreased compared with that in the control group. In Figs. 12C and 12F, n = 3-4 in each group. Error bars show SEMs. **, $P$ < 0.01 and ***, $P$ < 0.001 in one tailed unpaired t test.

Fig. 12D shows percent change in tumor volumes for individual animals in the control and treated (FOLFOX) mice from the start (day 1) to the end (day 22) of treatment.

Fig. 12E shows CT images of the patient before (d) and after (e) the irinotecan + cetuximab (C-mab) treatment. White arrowheads point metastatic lesions. The best response to irinotecan + cetuximab treatment in the patient was SD [(stable disease; +3% with RECIST (during d to e).

Fig. 12F shows results of drug-dosing test with PDSXs. The tumor growth in the treated (Irinotecan + C-mab) mice was significantly reduced compared with that in the control mice, and the tumor volume in the treated group appeared essentially unchanged.

Fig. 12G shows percent change in tumor volumes for individual animals in the control and treated (irinotecan + C-mab) mice from the start (day 1) to the end (day 22) of treatment.

Fig. 13A shows the serum CEA levels of a patient and chemotherapies given after resection of tumor (HC6T). The black diamond with (a) indicates an interruption of the chemotherapy for surgical resection of the primary lesion. The timing of CT scans (shown in Fig. 13B) is indicated as "b-d" at the bottom.

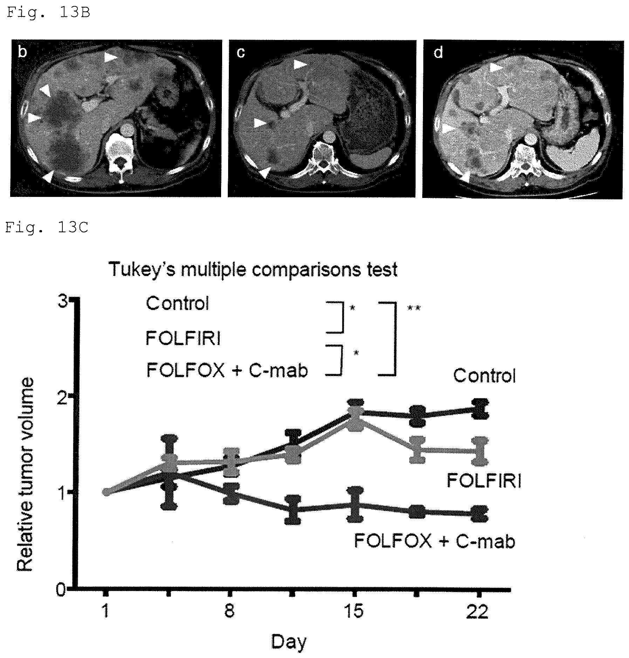

Fig. 13B shows CT images of the patient before (b) and after FOLFOX + panitumumab (P-mab) treatment (c), and followed by FOLFIRI + bevacizumab (B-mab) treatment (d). The best response to the SOX treatment in the patient was SD (stable disease; +11% in RECIST). The best response to the FOLFOX + P-mab treatment in the patient was PR (partial response; -60% with RECIST) after six months of the treatment. The best response to the FOLFIRI + B-mab treatment in the patient was SD (stable disease; +10% in RECIST) after three months of the treatment. Metastatic lesions increased in size rapidly after six months of the FOLFOX + P-mab treatment (d).

Fig. 13C shows results of drug dosing test with PDSXs derived from HC6T. The tumor size in the FOLFOX + cetuximab mice was reduced significantly compared with that in the control mice. On the other hand, the tumor size in the FOLFIRI mice decreased only slightly compared with that in the control mice. n = 2-3 in each group. Error bars show SEM. *, $P < 0.05$; **, and $P < 0.01$ in Tukey's multiple comparisons test.

Fig. 13D shows percent change in tumor volumes for individual animals in the control and treated mice from the start (day 1) to the end (day 22) of treatment.

Fig. 14A shows representative electropherograms of an MSS (microsatellite stable) case for five microsatellite markers of the Bethesda panel. In Figs. 14A-14C, shown are results of PCR products from normal cells and cancer cells.

Fig. 14B shows representative electropherograms of an MSI-H case for five microsatellite markers of the Bethesda panel.

Fig. 14C shows representative electropherograms of an MSI-H case for five microsatellite markers of the Bethesda panel.

Fig. 14D shows immunohistochemistry for mismatch repair (MMR) proteins (MLH1, MSH2, MSH6 and PMS2). The upper panels show primary lesion and spheroids from an MMR-proficient case. Expression of all proteins was confirmed. The lower panels show primary lesion and spheroids from an MMR-deficient case. Expression of MLH1 and PMS was not detected.

Fig. 14E shows sequence chromatograms containing the mononucleotide repeat region using spheroid-derived DNA samples. Left, TGFBR2 (reverse strand), right, BAX (reverse strand). The wild type (top), homozygous mutations (middle) and heterozygous mutations (bottom) are shown respectively.

Fig. 15A shows concentration and purity of DNA samples extracted from spheroids and formalin-fixed and paraffin-embedded (FFPE) tissues (left, concentration; right, A260/A80)

Fig. 15B shows maximum peak height in electoropherograms corresponding to microsatellite markers as determined by the fluorescence intensity in spheroid- and FFPE tissue-derived DNA samples. Spheroid-derived DNA samples gave taller peaks than those from FFPE-derived DNA samples.

Fig. 15C shows mutation profiles for eight MSI cases analyzed with spheroid- and FFPE tissue- derived DNA samples. Gray columns indicate mutated alleles, whereas white ones indicate wild-type alleles. Analysis with spheroid-derived DNA samples enabled identification of both alleles, while FFPE tissue-derived DNA samples gave confusing results.

Fig. 15D shows results of sequencing of a homozygous mutation case in BAX (G7/G7). Left, spheroid-derived DNA samples; right, FFPE tissue-derived DNA samples. With FFPE tissue-derived DNA samples, a slightly low peak for the 8th G was detected. This was derived from DNA of normal (i.e., wild-type) cells.

Fig. 16A shows dose-dependent effects (top) and computer-fitted dose-response curves (bottom) of five FGFR inhibitors on the growth of a colorectal cancer patient-derived spheroid HC6T. The data at the concentration of $10^{-5}$ M (10 $\mu$M) were excluded from the computation of the dose-response curves because of their non-specific nature of the toxicity.

Fig. 16B shows dose-dependent effects (top) and computer-fitted dose-response curves (bottom) of five FGFR inhibitors on the growth of HC20T.

Fig. 16C shows dose-dependent effects (top) and computer-fitted dose-response curves (bottom) of five FGFR inhibitors on the growth of HC9T.

Fig. 16D shows effects of erdafitinib on the growth of *RAS/RAF* wild type (wt) spheroid lines, HC1T, HC10T, HC11T and HC73T.

Fig. 16E shows effects of erdafitinib on the growth of *RAS/RAF* mutant (mt) spheroid lines, HC5T and HC13T.

Fig. 16F shows effects of erdafitinib on the growth of normal colonic epithelial spheroids.

Fig. 17A shows effects of erlotinib (left) or cetuximab (right) monotherapy on the growth of HC6T, HC9T, HC11T, and HC20T.

Fig. 17B shows dose-dependent inhibitory effects of erdafitinib in the absence or presence of erlotinib (0.1, 1, and 10 $\mu$M) on the growth of HC6T, HC20T, and HC9T.

Fig. 17C shows effects of erlotinib alone (1 $\mu$M), erdafitinib alone (0.01 $\mu$M), and combination of both on the growth of spheroid lines that were free of *RAS/RAF* hot-spot mutations (HC1T, HC7T, HC8T, HC10T, HC11T, HC21T and HC73T). *, $P < 0.05$-0.0001.

Fig. 18A shows effects of erdafitinib and/or cetuximab on tumor volume in HC6T-grafted PDXS mice. *, $P < 0.05$; **, $P < 0.01$; ***, $P < 0.001$; or ****, $P < 0.0001$.

Fig. 18B shows effects of erdafitinib and/or cetuximab on tumor volume in HC20T-grafted PDXS mice. *, $P < 0.05$.

Fig. 18C shows histological specimens of HC20T-PDSX tumors at day 21 stained with H&E. Scale bars, 100 $\mu$m or 50 $\mu$m (inset).

Fig. 18D shows histological specimens of HC20T-PDSX tumors at day 21 immunostained for Ki-67. Scale bars, 100 $\mu$m or 50 $\mu$m (inset).

Fig. 18E shows histological specimens of HC6T-PDSX tumors at day 21 stained with H&E. Scale bars, 100 $\mu$m or

50 $\mu$m (inset).

Fig. 18F shows histological specimens of HC6T-PDSX tumors at day 21 immunostained for Ki-67. Scale bars, 100 $\mu$m or 50 $\mu$m (inset).

Fig. 18G shows the fraction of proliferating cells (%) monitored by Ki67 expression for each treatment group. One of three tumors in the erdafitinib monotherapy group disappeared (complete response), and this case was excluded from the figure. *, $P < 0.05$.

Fig. 19 shows changes in tumor volume of PDSX mice in a drug sensitivity test (from the date of spheroid transplantation to the start of the test) (left, HC6T; right, HC20T).

Fig. 20 shows FGF or FGFR mRNA expression levels in patient-derived colorectal cancer spheroids.

Detailed Description of Embodiments

1. Definitions

Unless otherwise defined, the terms used herein are read as generally understood by a skilled person in the technical fields such as organic chemistry, medical sciences, pharmaceutical sciences, molecular biology, and microbiology. Several terms used herein are defined as described below. The definitions herein take precedence over the general understanding.

When a numerical value is accompanied with the term "about", the value is intended to represent any value in the range of -10% of the value to +10% of the value. A range defined with a value of the lower limit and a value of the upper limit covers all values from the lower limit to the upper limit, including the values of both limits. When a range is accompanied with the term "about", both limits are read as accompanied with the term. For example, "about 20 to 30" is read as "$20\pm10\%$ to $30\pm10\%$."

As used herein, a cancer spheroid means a cell mass containing a tumor initiating cell. The tumor initiating cell means a cell being able to form a tumor when transplanted into an immunodeficient animal. In general, a cancer spheroid contains about 100 cells to about 10,000 cells, preferably about 500 cells to about 2000 cells, and has a shape being close to a sphere with a diameter of about 0.01 mm to about 2 mm, preferably about 0.1 mm to about 0.5 mm. Here, the "diameter" means the length of the longest axis of a cell mass.

The "cell population obtained from a cancer tissue of a patient" as used herein can be prepared by a conventional method. For example, the methods described in Miyoshi H, Stappenbeck TS. Nat Protoc. 2013; 8: 2471-82 or VanDussen KL, et al., Gut. 2015; 64: 911-20 may be used. In an embodiment, the cell population obtained from a cancer tissue of a patient is a cell population obtained by subjecting a cancer tissue obtained from a patient or an epithelial tissue separated from such a cancer tissue to an enzyme treatment (for example, collagenase treatment). The cell population obtained from a cancer tissue of a patient comprises a cancer cell and a tumor initiating cell.

In general, cells are obtained from a cancer tissue in a volume of about 0.1 cm$^3$ to 5.0 cm$^3$, about 0.2 cm$^3$ to 3.0 cm$^3$, or about 0.5 cm$^3$ to 2.0 cm$^3$, preferably about 0.5 cm$^3$ to 1.0 cm$^3$, and cultured. The cells are preferably cultured with a basement membrane matrix such as Matrigel® (Corning) or Cultrex® Basement Membrane Extract (Trevigen). For example, 10$^3$ cells to 10$^5$ cells are mixed with a basement membrane matrix, and the cell suspension thus obtained is seeded on a culture vessel (such as culture plate or culture dish). After the basement membrane matrix is solidified at 37°C, a culture solution is overlaid to start cell culture.

2. Method for preparing cancer spheroid and use of cancer spheroid

In an aspect, the present disclosure relates to a method of preparing a cancer spheroid, comprising culturing a cell population obtained from a cancer tissue of a patient in a medium containing

a ROCK inhibitor, a TGF-$\beta$ type I receptor inhibitor, EGF and bFGF; and

an agent selected from an adenosine receptor agonist, a PPAR agonist, a calcium-activated potassium-channel (KCa) activator, an NMDA-type glutamate receptor agonist, a histamine H3 receptor agonist, and a retinoic acid receptor (RXR) $\beta$2 agonist.

Examples of cancers include colorectal cancer, gastric cancer, prostate cancer, breast cancer, cervical cancer, ovarian cancer, lung cancer, hepatocellular cancer, kidney cancer, pancreatic cancer, and bladder cancer. Preferably, the cancer is colorectal cancer. The cancer tissue may be a tissue obtained from a primary lesion or a metastatic lesion, but is preferably a tissue obtained from a primary lesion. The cancer tissue may be a surgical specimen or a biopsy specimen.

The medium may be any medium commonly used for spheroid culture, and examples thereof include Advanced DMEM/F-12 (Thermo Fisher) and DMEM/F12 (Thermo Fisher) supplemented with ITS supplement (Thermo Fisher).

Examples of adenosine receptor agonists include NECA (5'-(N-ethyl-carboxamido)-adenosine), CV1808 (2-phenylaminoadenosine), and BAY 60-6583 (2-[[6-Amino-3,5-dicyano-4-[4-(cyclopropylmethoxy)phenyl]-2-pyridinyl]thio]-acetamide). Preferably, the adenosine receptor agonist is NECA or CV1808, and more preferably NECA. The adenosine receptor agonist may be used, for example, at a concentration of 0.001 $\mu$M to 10 $\mu$M, preferably 0.1 $\mu$M to 5 $\mu$M, more preferably 0.1 $\mu$M to 1 $\mu$M.

The PPAR agonist includes a PPAR$\alpha$ agonist, a PPAR$\gamma$ agonist and a PPAR$\delta$ agonist.

Examples of PPAR$\alpha$ agonists include GW7647 (2-methyl-2-[[4-2-[[(cyclohexylamino)carbonyl](4-cyclohexylbutyl)amino]ethyl]phenyl]thio]-propanoic acid) and CP 775146 (2-Methyl-2-[3-[(3S)-1-[2-[4-(1-methylethyl)phenyl]acetyl]-3-piperidinyl]phenoxy]-propanoic acid);

Examples of PPAR$\gamma$ agonists include S26948 ([[4-[2-(6-Benzoyl-2-oxo-3(2H)-benzothiazolyl)ethoxy]phenyl]methyl]-1,3-propanedioic acid dimethyl ester) and Troglitazone (5-[[4-[(3,4-Dihydro-6-hydroxy-2,5,7,8-tetramethyl-2*H*-1-benzopyran-2-yl)methoxy]phenyl]methyl]-2,4-thiazolidinedione);

Examples of PPAR$\delta$ agonists include GW0742 ([4-[[[2-[3-fluoro-4-(trifluoromethyl)phenyl]-4-methyl-5-thiazolyl]methyl]thio]-2-methyl phenoxy]-acetic acid), L-165,041 ([4-[3-(4-Acetyl-3-hydroxy-2-propylphenoxy)propoxy]phenoxy]acetic acid), and GW 501516 (2-[2-Methyl-4-[[[4-methyl-2-[4-(trifluoromethyl)phenyl]-5-thiazolyl]methyl]thio]phenoxy]acetic acid).

Examples of KCa activators include DCEBIO (5,6-Dichloro-1-ethyl-1,3-dihydro-2H-benzimidazol-2-one), SKA-31 (Naphtho[1,2-d]thiazol-2-ylamine), and 1-EBIO (1-Ethyl-2-benzimidazolinone). Preferably, the KCa activator is DCEBIO or SKA-31. The KCa activator may be used, for example, at a concentration of 0.1 $\mu$M to 50 $\mu$M, preferably 1 $\mu$M to 30 $\mu$M, more preferably about 10 $\mu$M.

Examples of NMDA-type glutamate receptor agonists include NMDA (N-Methyl-D-aspartic acid) and Homoquinolinic acid. Preferably, the NMDA-type glutamate receptor agonist is NMDA. The NMDA-type glutamate receptor agonist may be used at a concentration of, for example, 0.1 $\mu$M to 50 $\mu$M, preferably 1 $\mu$M to 30 $\mu$M, more preferably about 10 $\mu$M.

Examples of histamine H3 receptor agonists include Immepip (4-(1H-Imidazol-4-ylmethyl)piperidine) and Immethridine (4-(1H-Imidazol-4-ylmethyl)pyridine). Preferably, the histamine H3 receptor agonist is Immepip. The histamine H3 receptor agonist may be used at a concentration of, for example, 0.1 $\mu$M to 50 $\mu$M, preferably 1 $\mu$M to 30 $\mu$M, more preferably about 10 $\mu$M.

Examples of RXR$\beta$2 agonists include AC55649 (4'-Octyl-[1,1'-biphenyl]-4-carboxylic acid) and AC 261066 (4-[4-(2-Butoxyethoxy-)-5-methyl-2-thiazolyl]-2-fluorobenzoic acid). Preferably, the RXR$\beta$2 agonist is AC55649. The RXR$\beta$2 agonist may be used, for example, at a concentration of 0.1 $\mu$M to 50 $\mu$M, preferably 1 $\mu$M to 30 $\mu$M, more preferably about 10 $\mu$M.

Examples of ROCK inhibitors include Y27632 ((R)-(+)-trans-4-(1-Aminoethyl)-N-(4-pyridyl)cyclohexanecarboxamide) and H1152 ((S)-(+)-2-Methyl-1-[(4-methyl-5-isoquinolinyl)sulfonyl]-hexahydro-1H-1,4-diazepine). Preferably the ROCK inhibitor is Y27632. The ROCK inhibitor may be used, for example, at a concentration of 0.1 $\mu$M to 50 $\mu$M, preferably 1 $\mu$M to 30 $\mu$M, more preferably about 10 $\mu$M.

Examples of TGF-$\beta$ (transforming growth factor-$\beta$) type I receptor inhibitors include SB431542 (4-[4-(1,3-benzodioxol-5-yl)-5-(2-pyridinyl)-1H-imidazol-2-yl]benzamide) and A83-01 (3-(6-Methyl-2-pyridinyl)-N-phenyl-4-(4-quinolinyl)-1H-pyrazole-1-carbothioamide). Preferably, the TGF-$\beta$ type I receptor inhibitor is SB431542. The TGF-$\beta$ type I receptor inhibitor may be used, for example, at a concentration of 0.01 $\mu$M to 10 $\mu$M, preferably 0.1 $\mu$M to 10 $\mu$M, more preferably 0.5 $\mu$M to 10 $\mu$M.

EGF (epidermal growth factor) and bFGF (basic fibroblast growth factor) may be a recombinant human protein. EGF may be used at a concentration of, for example, 10 ng/ml to 100 ng/ml, preferably about 50 ng/ml. bFGF may be used at a concentration of, for example, 50 ng/ml to 200 ng/ml, preferably about 100 ng/ml.

The medium usually contains serum (such as fetal bovine serum (FBS)). The serum may be used at a concentration of about 1-10%, preferably about 5%.

The medium may contain two or more agents selected from an adenosine receptor agonist, a PPAR agonist, a KCa activator, an NMDA-type glutamate receptor agonist, a histamine H3 receptor agonist, and a RXR $\beta$2 agonist.

The medium may further contain an antibiotic such as penicillin, streptomycin, or plasmin, L-glutamine, or B27 supplement (Thermo Fisher).

The period of culturing cells is not particularly limited to a specific period, and the culture may be continued until an appropriate number of cancer spheroid cells are obtained. In general, the culture is continued until about $1 \times 10^5$ to about $1 \times 10^7$, preferably about $10^6$ cancer spheroid cells are obtained. The culture period may be, for example, one week to two months (for example, one week to four weeks, or one month to two months). During the culture period, the medium is preferably replaced with a fresh medium every other day or every few days (for example, two or three days). Usually, during the continuous culture, a procedure of treating the formed cell mass with an enzyme (such as trypsin) to separate cells and re-forming a cell mass is performed once or more.

The cancer spheroid thus obtained may be further cultured for several days to several weeks until the number of cells becomes an appropriate number depending on the purpose of use. Also, the cancer spheroid may be stored frozen until use, and may be cultured until an appropriate cell number is obtained after thawing. The cancer spheroid may be passaged during the culture. As used herein, a cancer spheroid derived from the cancer spheroid thus obtained means a cancer spheroid obtained by passaging a cancer spheroid prepared by the method of the present disclosure. The passaging may or may not involve treating the cancer spheroid with an enzyme (such as trypsin) to separate cells and re-forming a cancer spheroid.

A cancer spheroid may be provided as a composition comprising the cancer spheroid and a solution suitable for culturing or storing the cancer spheroid. The composition comprising a cancer spheroid may be frozen, in which case the composition may comprise a cancer spheroid and a suitable cryopreservation liquid.

A cancer spheroid can be used, for example, for *in vivo* or *in vitro* drug sensitivity evaluation, drug screening, and microsatellite instability (MSI) test. A cancer spheroid can also be used for searching for a biomarker of drug sensitivity based on the results of drug sensitivity evaluation. The results of drug sensitivity evaluation can be used not only for selection of treatment for patients, but also for selection of clinical trial participants in new drug development.

A reporter gene may be introduced into a cell in a cancer spheroid to monitor cell proliferation. Examples of reporter genes include luciferase and green fluorescent protein (GFP). The reporter gene can be introduced using a vector such as a plasmid vector or a viral vector. In an embodiment, the reporter gene is introduced into a cell in a cancer spheroid by a viral vector such as a retroviral vector, a lentiviral vector, an adenoviral vector, or an adeno-associated viral vector, preferably by a lentiviral vector. The vector can be introduced by a conventional method, for example, the methods described in the example section of the present disclosure or Miyoshi H, Stappenbeck TS. Nat Protoc. 2013; 8: 2471-82. Introduction of the reporter gene is preferably performed after a cancer spheroid is treated with an enzyme (such as trypsin) to separate cells. After the reporter gene is introduced, a spheroid can be re-formed and used for an assay.

A patient-derived spheroid xenograft (PDSX) model can be prepared by transplanting a cancer spheroid into a non-human animal. Examples of non-human animals include immunodeficient animals such as nude mice, nude rats, SCID mice, NOD-SCID mice, and NOG mice. The transplantation site is not particularly limited to a specific site, but subcutaneous transplantation is preferred. The number of cells in a cancer spheroid to be transplanted may be any number, but in the case of a mouse, it is usually about $1 \times 10^5$ to about $9 \times 10^5$, preferably about $5 \times 10^5$. It is preferable that the non-human animal is used for a test when the tumor volume reaches about 300-500 mm$^3$ after the transplantation.

By administering a candidate agent to a PDSX model and determining response to the candidate agent, drug sensitivity of a patient from whom a cancer spheroid is derived can be evaluated, or drug screening can be carried out. The response of cells can be determined based on the volume or a histological feature of a tumor tissue. Examples of histological features include the number of mitotic figures and the number of degenerated cells. For example, when an increase in the volume of a tumor tissue is significantly suppressed, or when a histological feature is improved (for example, when the number of mitotic figures is decreased or the number of degenerated cells is increased) as compared to a control PDSX model, to which the candidate agent has not been administered, the patient from whom the cancer spheroid is derived is evaluated as being sensitive to the candidate agent, or the candidate agent is evaluated as being effective in treating cancer.

Examples of candidate agents include, but not limited to, single compounds such as low molecular weight compounds, proteins, antibodies, peptides, nucleic acids; libraries of compounds, antibodies, nucleic acids, or other components; or cell extracts, cell culture supernatants, microorganism fermentation products, marine organism extracts, and plant extracts. When drug sensitivity of a patient is evaluated, the candidate agent may be a known drug for cancer therapy.

Drug sensitivity evaluation and drug screening can also be carried out by contacting a cancer spheroid with a candidate agent *in vitro* and determining response to the candidate agent. For example, when the candidate agent significantly suppresses an increase in the volume of a cancer spheroid or improves a histological feature of a cancer spheroid compared to a control cancer spheroid, which has not been contacted with the candidate agent, the patient is evaluated as being sensitive to the candidate agent, or the candidate agent is evaluated as being effective in treating cancer.

MSI is a phenomenon in which a microsatellite shows a different number of repeats in a tumor tissue as compared to a non-tumor tissue due to dysfunction of a mismatch repair gene. The MSI test is known to be useful as an adjunctive diagnosis of Lynch syndrome, which is caused by germline mutations of mismatch repair genes, or in predicting effects of an immune checkpoint inhibitor (such as anti-PD-1 antibody) on colorectal cancer. The MSI test usually examines the number of microsatellite repeats in a tumor tissue and a non-tumor tissue, and a cancer spheroid can be used as a tumor tissue in place of a surgical or biopsy specimen. The use of a cancer spheroid enables more accurate examination.

The number of microsatellite repeats can be determined by a conventional method. For example, a region containing a microsatellite repeat sequence is amplified by PCR from DNA obtained from a cancer spheroid and a non-tumor tissue (or blood when it is difficult to collect a non-tumor tissue), and the number of repeats of the microsatellite is compared. The result is MSI-positive when the number of microsatellite repeats in the cancer spheroid is changed compared to the non-tumor tissue. The MSI status is usually determined using five microsatellite markers of BAT25, BAT26, D5S346, D2S123, and D17S250 (Bethesda Panel). The MSI status is determined as MSI-H (MSI-high) when two or more of the markers show MSI, as MSI-L (MSI-low) when one of the markers shows MSI, and as MSS (microsatellite stable) when MSI was not observed. When one or more additional markers are used in addition to the five makers, the MSI status is determined as MSI-H when 30-40% or more of the markers used shows MSI, and as MSI-L when less than 30% of the markers does.

The MSI test can also be carried out by examining a mutation in a target gene of a mismatch repair gene, which is a gene showing abnormality due to functional abnormality of the mismatch repair gene. Examples of target genes include TGFBR2, IGF2R, BAX, and CASP5. The mutation can be examined by using DNA obtained from a cancer spheroid, and a conventional method for detecting a gene mutation (for example, a nucleic acid hybridization method using a DNA chip or a DNA microarray, direct sequence method, TaqMan PCR method, RFLP method, or PCR-SSCP method). DNA obtained from a cancer spheroid has a higher purity than that from a surgical or biopsy specimen and enables detection of a heterozygous mutation that cannot be detected previously, and thus enables an accurate examination.

3. Method of selecting colorectal cancer patient, pharmaceutical composition for treating colorectal cancer, and method for treating colorectal cancer

In an aspect, the present disclosure provides a method of selecting a colorectal cancer patient being sensitive to an FGFR inhibitor, comprising
treating a cancer spheroid or a patient-derived spheroid xenograft (PDSX) model derived from a colorectal cancer patient with an FGFR inhibitor, and
determining response to the FGFR inhibitor of the cancer spheroid or the PDSX model, and
selecting the colorectal cancer patient when the response to the FGFR inhibitor is positive.

The large intestine is divided into the colon and the rectum, and the term "colorectal cancer" in the present disclosure includes colon cancer and rectal cancer. Colorectal cancer is divided into stages 0, I, II, III, and IV according to the degree of progression, and the term "colorectal cancer" in the present disclosure includes any stage of cancer.

As used herein, the term "fibroblast growth factor receptor (FGFR) inhibitor" means a substance that inhibits signal transduction mediated by FGFR. There are four types of FGFR, FGFR1, FGFR2, FGFR3, and FGFR4, and an FGFR inhibitor can inhibit one or more of these four types of FGFR. In an embodiment, the FGFR inhibitor inhibits three types of FGFR1, FGFR2, and FGFR3, or four types of FGFR1, FGFR2, FGFR3, and FGFR4. The FGFR inhibitor can be a small molecule compound, a protein, an antibody, a peptide, or a nucleic acid. Examples of FGFR inhibitors include erdafitinib (JNJ42756493), rogolatinib (BAY1163877), AZD4547, BGJ398, BLU554, LY2874455, ASP5878 and ARQ087. In an embodiment, the FGFR inhibitor is erdafitinib.

As used herein, the term "cancer spheroid derived from a colorectal cancer patient" includes a cancer spheroid prepared from a cell population obtained from a cancer tissue of a colorectal cancer patient, and a cancer spheroid obtained by passaging such a cancer spheroid. The cancer tissue may be a tissue obtained from a primary lesion or a metastatic lesion, but is preferably a tissue obtained from a primary lesion. The cancer tissue can be a surgical specimen or a biopsy specimen. As used herein, "a PDSX model derived from a colorectal cancer patient" is a model animal prepared by transplanting a cancer spheroid derived from a colorectal cancer patient into a non-human animal.

In this aspect, the cancer spheroid may be prepared by any method from a cell population obtained from a cancer tissue of a patient. For example, the methods described in Non-Patent Documents 1 to 7 of the present disclosure can be used.

In an embodiment, the cancer spheroid is prepared by a method that comprises culturing a cell population obtained from a cancer tissue of a patient in a medium containing a ROCK inhibitor, a TGF-β type I receptor inhibitor, EGF and bFGF. In this embodiment, the cancer spheroid can be prepared according to the descriptions of item 2 above, except that the medium in this embodiment does not necessarily contain an agent selected from an adenosine receptor agonist, a PPAR agonist, a KCa activator, an NMDA-type glutamate receptor agonist, a histamine H3 receptor agonist, and a RXR β2 agonist.

In a further embodiment, the cancer spheroid is prepared by the method for preparing a cancer spheroid of the present disclosure as described in item 2 above. The method for preparing a cancer spheroid of the present disclosure enables efficient preparation of a cancer spheroid.

By treating a cancer spheroid or PDSX model derived from a colorectal cancer patient with an FGFR inhibitor and determining response of the cancer spheroid or PDSX model to the FGFR inhibitor, sensitivity of the colorectal cancer patient to the FGFR inhibitor can be evaluated. When the response to the FGFR inhibitor is positive, the patient is evaluated as sensitive to the FGFR inhibitor and can be selected for the treatment with the FGFR inhibitor.

In the present disclosure, a cancer spheroid is treated with an FGFR inhibitor *in vitro.* An FGFR inhibitor is typically added to the culture medium of the cancer spheroid. A cancer spheroid is preferably cultured with a basement membrane matrix such as Matrigel® or Cultrex® Basement Membrane Extract. For example, a cancer spheroid derived from a colorectal cancer patient is treated with an enzyme (such as trypsin) to separate cells, mixed with a basement membrane matrix at about $5 \times 10^4$ to $2 \times 10^6$ cells/ml, and the resulting cell suspension is seeded on a culture vessel (such as culture plate or culture dish). After the basement membrane matrix is solidified at 37°C, a medium is overlaid for culture. The medium may be any medium commonly used for spheroid culture as described in item 2 above. An FGFR inhibitor is added at a concentration that is appropriately determined depending on the FGFR inhibitor to be evaluated. For example, an FGFR inhibitor may be added at final concentration of $10^{-10}$ M to $10^{-5}$ M or $10^{-9}$ M to $10^{-6}$ M. After a period of time from the start of the treatment with the FGFR inhibitor, for example, 1 day, 2 days, 3 days, 4 days, 5 days, 6 days, 7 days, 8 days, 9 days, or 10 days later, the response to the FGFR inhibitor is determined.

In the present disclosure, the PDSX model is a non-human animal. Examples of non-human animals for preparing a PDSX model include immunodeficient animals such as nude mice, nude rats, SCID mice, NOD-SCID mice and NOG mice. The transplantation site is not particularly limited to a specific site, but subcutaneous transplantation is preferred. The number of cells in a cancer spheroid to be transplanted may be any number, but in the case of a mouse, it is usually about $1 \times 10^5$ to about $9 \times 10^5$, preferably about $5 \times 10^5$. It is preferable that the non-human animal is used for a test when the tumor volume reaches about 300-500 mm$^3$ after the transplantation. An FGFR inhibitor is administered at a dose appropriately determined according to the FGFR inhibitor to be evaluated. For example, an FGFR inhibitor is administered at 0.1 mg/kg body weight to 100 mg/kg body weight, 1 mg/kg body weight to 30 mg/kg body weight, or 10 mg/kg to 30 mg/kg body weight. An FGFR inhibitor is administered at an appropriately determined administration interval, and may be administered once or multiple times a day, which may be performed every day or every few days, or every week or every few weeks. The response to the FGFR inhibitor is determined after a period of time, for example one week to several months, for example one week, two weeks, three weeks or four weeks after the start of the treatment with the FGFR inhibitor.

The response of a cancer spheroid to an FGFR inhibitor can be determined based on, for example, growth or a histological feature of a cancer spheroid. The growth of a cancer spheroid can be determined by measuring a signal (for example, fluorescence) from a reporter gene contained in a cell of the cancer spheroid. The response of a PDSX model to an FGFR inhibitor can be determined based on the volume or a histological feature of a tumor tissue that is derived from the transplanted cancer spheroid. The volume of a tumor tissue can be calculated by the following formula: tumor volume (mm$^3$) = [length (mm) $\times$ width$^2$ (mm$^2$)] / 2 (wherein the length means the longer tumor axis and the width means the shorter tumor axis). The histological feature of a cancer spheroid or a tumor tissue can be examined by hematoxylin/eosin staining (H&E staining) or immunostaining for a cell proliferation marker (such as Ki67, PCNA, or MCM2). Histological features include the number of mitotic figures and the number of degenerated cells.

The response to an FGFR inhibitor is determined by comparing a value obtained from a cancer spheroid or PDSX model treated with the FGFR inhibitor with a reference value. In an embodiment, the response to an FGFR inhibitor is determined to be positive when the value obtained from a cancer spheroid or PDSX model treated with the FGFR inhibitor is lower than a reference value. The reference value may be a value obtained from a cancer spheroid or PDSX model that has not been treated with the FGFR inhibitor (control value) . Alternatively, the reference value may be 90% to 10% (such as 90%, 80%, 70%, 60%, 50%, 40%, 30%, 20%, or 10%) of the control value, for example 90% to 50% of the control value. For example, when the growth of the cancer spheroid treated with an FGFR inhibitor, the volume of a tumor tissue of the PDSX model treated with an FGFR inhibitor, or the number of mitotic figures in the cancer spheroid or PDSX model is lower than the reference value, the response to the FGFR inhibitor is determined to be positive.

In a different embodiment, the response to an FGFR inhibitor is determined to be positive when the value obtained from a cancer spheroid or PDSX model treated with the FGFR inhibitor is higher than a reference value. The reference value may be the control value. Alternatively, the reference value may be 110-200% (such as 110%, 120%, 130%, 140%, 150%, 160%, 170%, 180%, 190%, or 200%) of the control value, for example 110%-150% of the control value. For example, when the number of degenerated cells in the cancer spheroid or PDSX model treated with a FGFR inhibitor is higher than the reference value, the response to the FGFR inhibitor is determined to be positive.

The comparison with a reference value may or may not include a statistical analysis. Examples of statistical analysis methods include one-way analysis of variance (one-way ANOVA), two-way analysis of variance (two-way ANOVA), Dunnett's test, and Tukey's test.

In an embodiment, the response of a cancer spheroid to an FGFR inhibitor is determined based on the growth of the cancer spheroid, and the reference value to be used is 90%-50%, for example 70%, of the control value. When the growth of the cancer spheroid treated with an FGFR inhibitor is lower than the reference value, the response to the FGFR inhibitor is determined to be positive.

In a different embodiment, the response to an FGFR inhibitor of a PDSX model is determined based on the volume of a tumor tissue of the PDSX model, and the reference value to be used is the control value. When the volume of a tumor tissue of the PDSX model treated with an FGFR inhibitor is statistically significantly lower than the reference value, the response to the FGFR inhibitor is determined to be positive

As shown in the example section, it was not possible to select a colorectal cancer patient being sensitive to an FGFR inhibitor based on the copy number or mRNA amount of the FGFR gene, which has been used as a patient selection criterion for an FGFR inhibitor. The patient selection method of the present disclosure enables selection of a colorectal cancer patient who is sensitive to an FGFR inhibitor, and expands the application of FGFR inhibitors.

The method of the present disclosure may include, in addition to the response to an FGFR inhibitor, determining the response to a combination of the FGFR inhibitor and one or more agents different from the FGFR inhibitor. When the one or more agents enhance the response to the FGFR inhibitor, the agent (s) can be used with the FGFR inhibitor in the treatment of colorectal cancer. The one or more agents may be those exemplified herein as agents that may be used in combination with an FGFR inhibitor in the treatment of colorectal cancer.

The treatment of colorectal cancer may be treatment before or after resection of a cancer tissue. For example, the treatment of colorectal cancer can be treatment of unresectable colorectal cancer or treatment to prevent recurrence after resection of a cancer tissue. For the treatment of colorectal cancer, an effective amount of an FGFR inhibitor is administered to a colorectal cancer patient. As used herein, "an effective amount" is an amount capable of exerting a desired effect in the treatment of colorectal cancer, such as elimination or shrinkage of tumor, suppression of tumor growth, or prevention of recurrence. An FGFR inhibitor is administered at a dose appropriately determined according to the age or body weight of the patient, severity of the disease, concomitant drug or other factors, and may be administered, for example, at 0.01 mg/kg body weight to 100 mg/kg body weight, 0.1 mg/kg body weight to 100 mg/kg body weight, 0.1 mg/kg body weight to 10 mg/kg body weight, or 0.1 mg/kg body weight to 1 mg/kg body weight per day. In an embodiment, the FGFR inhibitor is erdafitinib and administered at 1 mg/day to 30 mg/day, 1 mg/day to 15 mg/day, 1 mg/day to 12 mg/day, or 1 mg/day to 10 mg/day. An FGFR inhibitor can be administered orally or parenterally (for example, by intravenous administration, intramuscular administration, or subcutaneous administration). An FGFR inhibitor may be administered once, twice, three times, four times or more times a day, and may be administered daily or at regular intervals.

An FGFR inhibitor may be combined with endoscopic resection, surgical resection, or radiation therapy for the treatment of colorectal cancer, or may be combined with one or more agents in chemotherapy. The one or more agents can be selected from, for example, anti-cancer agents such as 5-fluorouracil (5-FU), tegafur, tegafur-uracil (UFT), doxyfluridine (5'-DFUR), carmofur, tegafur-guimeracil-oteracil potassium (S-1), capecitabine (Cape), regorafenib, trifluridine-tipiracil hydrochloride (TAS-102), mitomycin C, irinotecan (IRI), and oxaliplatin (OX), and molecular targeting agents such as vascular endothelial growth factor (VEGF) inhibitors (such as bevacizumab and aflibercept), vascular endothelial growth factor receptor (VEGFR) inhibitors (such as ramucirumab), EGFR inhibitors (such as cetuximab, panitumumab, or erlotinib). In an embodiment, an FGFR inhibitor is combined with an EGFR inhibitor. In an embodiment, the EGFR inhibitor is cetuximab or panitumumab. In a further embodiment, the EGFR inhibitor is cetuximab. When it is described herein that an FGFR inhibitor is used in combination with one or more agents, it also includes the case where these are used in combination with one or more additional agents.

In an embodiment, an FGFR inhibitor is used in combination with a chemotherapy such as FOLFOX therapy (drip 5-FU + levofolinate + OX), FOLFIRI therapy (5-FU + levofolinate + IRI), FOLFOXIRI therapy (5-FU + levofolinate + OX + IRI), CapeOX therapy (Cape + OX), SOX therapy (S-1 + OX), or IRIS therapy (S-1 + IRI), or a combination of such a chemotherapy and one or more molecular targeting agents. In an embodiment, the one or more molecular targeting agents include an EGFR inhibitor. In an embodiment, the EGFR inhibitor is cetuximab or panitumumab. In a further embodiment, the EGFR inhibitor is cetuximab.

When two or more agents are used in combination, some or all of these agents may be comprised in one composition, or all agents may be comprised in separate compositions. In addition, the administration schedule of these agents may be the same or different.

The pharmaceutical composition comprising an FGFR inhibitor can be formulated by a conventional method. The pharmaceutical composition may be in the form of tablets, capsules, powders, granules, suspensions, syrups, elixirs, or injections. Depending on the dosage form, the pharmaceutical composition may include, in addition to the FGFR inhibitor, a pharmaceutically acceptable additive selected from, for example, excipients, disintegrants, binders, lubricants, stabilizers, buffers, surfactants, preservatives, and sweeteners, and/or a pharmaceutically acceptable carrier selected from, for example, water, saline, oils, and alcohols.

Examples of embodiments of the present disclosure are provided below.

1. A method of preparing a cancer spheroid, comprising culturing a cell population obtained from a cancer tissue of a patient in a medium containing
a ROCK inhibitor, a TGF-β type I receptor inhibitor, EGF and bFGF; and
an agent selected from an adenosine receptor agonist, a PPAR agonist, a calcium-activated potassium-channel (KCa) activator, an NMDA-type glutamate receptor agonist, a histamine H3 receptor agonist, and a retinoic acid receptor (RXR) β2 agonist.

2. The method of item 1, wherein the cancer is colorectal cancer.

3. The method of item 1 or 2, wherein the cancer tissue is a tissue obtained from a primary lesion.

4. The method of any one of items 1-3, wherein the agent is an adenosine receptor agonist.

5. The method of item 4, wherein the adenosine receptor agonist is NECA or CV1808.

6. The method of item 5, wherein the adenosine receptor agonist is NECA.

7. The method of any one of items 1-3, wherein the agent is a PPAR agonist.

8. The method of item 7, wherein the PPAR agonist is GW0742, GW7647, S26948 or L-165,041.

9. The method of any one of items 1-3, wherein the agent is a KCa activator.

10. The method of item 9, wherein the KCa activator is DCEBIO or SKA-31.

11. The method of any one of items 1-3, wherein the agent is an NMDA-type glutamate receptor agonist.

12. The method of item 11, wherein the NMDA-type glutamate receptor agonist is NMDA.

13. The method of any one of items 1-3, wherein the agent is a histamine H3 receptor agonist.

14. The method of item 13, wherein the histamine H3 receptor agonist is imepip.

15. The method of any one of items 1-3, wherein the agent is a RXR $\beta$2 agonist.

16. The method of item 15, wherein the RXR $\beta$2 agonist is AC55649.

17. The method of any one of items 1-3, wherein the agent is selected from NECA, CV1808, GW0742, GW7647, S26948, L-165,041, DCEBIO, SKA-31, NMDA, imepip and AC55649.

18. The method of any one of items 1-17, wherein the ROCK inhibitor is Y27632.

19. The method of any one of items 1-18, wherein the TGF-$\beta$ type I receptor inhibitor is SB431542.

20. The method of any one of items 1-19, wherein the medium further comprises serum.

21. The method of any one of items 1-20, wherein the cell population is cultured for one week to two months.

22. A composition comprising a cancer spheroid obtained by the method of any one of items 1-21 or a cancer spheroid derived therefrom.

23. A method of evaluating drug sensitivity of a patient, comprising
preparing a cancer spheroid by the method of any one of items 1-21, and
contacting the cancer spheroid thus obtained or a cancer spheroid derived therefrom with a candidate agent *in vitro.*

24. A method of screening a therapeutic agent for a cancer, comprising
preparing a cancer spheroid by the method of any one of items 1-21, and
contacting the cancer spheroid thus obtained or a cancer spheroid derived therefrom with a candidate agent *in vitro.*

25. The method of item 23 or 24, wherein the method further comprises introducing a reporter gene into a cell within the cancer spheroid.

26. A method of preparing a patient-derived spheroid xenograft (PDSX) model, comprising
preparing a cancer spheroid by the method of any one of items 1-21, and
transplanting the cancer spheroid thus obtained or a cancer spheroid derived therefrom to a non-human animal.

27. A method of evaluating drug sensitivity of a patient, comprising

preparing a PDSX model by the method of item 26, and administering a candidate agent to the PDSX model thus obtained.

28. A method of screening a therapeutic agent for a cancer, comprising
preparing a PDSX model by the method of item 26, and administering a candidate agent to the PDSX model thus obtained.

29. A method of detecting microsatellite instability (MSI), comprising
preparing a cancer spheroid by the method of any one of items 1-21, and
determining MSI status or a mutation of a target gene of a mismatch repair gene in the cancer spheroid thus obtained or a cancer spheroid derived therefrom.

30. The method of item 29, wherein the method is for predicting efficacy of an immune checkpoint inhibitor.

31. The method of item 30, wherein the immune checkpoint inhibitor is an anti-PD-1 antibody.

32. The method of any one of items 29-31, wherein the determining comprises determining the number of repeats for each of microsatellite markers BAT25, BAT26, D5S346, D2S123, and D17S250.

33. The method of any one of items 29-31, wherein the target gene of a mismatch repair gene is TGFBR2, IGF2R, BAX, or CASP5.

Further examples of embodiments of the present disclosure are provided below.

1'. A method of selecting a colorectal cancer patient being sensitive to an FGFR inhibitor, comprising
treating a cancer spheroid or a patient-derived spheroid xenograft (PDSX) model derived from a colorectal cancer patient with an FGFR inhibitor, and
determining response to the FGFR inhibitor of the cancer spheroid or the PDSX model, and
selecting the colorectal cancer patient when the response to the FGFR inhibitor is positive.

2'. The method of item 1', wherein the treating comprises treating a cancer spheroid derived from a colorectal cancer patient with an FGFR inhibitor.

3'. The method of item 2', wherein the response to the FGFR inhibitor is determined based on growth or a histological feature of the cancer spheroid.

4' The method of item 1', wherein the treating comprises treating a PDSX model with an FGFR inhibitor.

5' The method of item 4', wherein the response to the FGFR inhibitor is determined based on the volume or a histological feature of a tumor tissue of the PDSX model.

6'. The method of any one of items 1'-5', wherein the method further comprises preparing the cancer spheroid from a cell population obtained from a cancer tissue of the colorectal cancer patient.

7'. The method of any one of items 1'-6', wherein the method further comprises preparing the PDXS model by transplanting a cancer spheroid derived from the colorectal cancer patient to a non-human animal.

8'. The method of any one of items 1'-7', wherein the FGFR inhibitor is selected from erdafitinib, rogoratinib, AZD4547, BGJ398, BLU554, LY2874455, ASP5878, and ARQ087.

9'. The method of any one of items 1'-8', wherein the FGFR inhibitor is erdafitinib.

10'. The method of any one of items 1'-9', wherein the cancer spheroid is a spheroid prepared by culturing a cell population obtained from a cancer tissue of the patient in a medium containing a ROCK inhibitor, a TGF-β type I receptor inhibitor, EGF and bFGF.

11'. The method of item 10', wherein the medium futher comprises an agent selected from an adenosine receptor agonist, a PPAR agonist, a calcium-activated potassium-channel (KCa) activator, an NMDA-type glutamate receptor agonist, a histamine H3 receptor agonist, and a retinoic acid receptor (RXR) β2 agonist.

12'. The method of item 11', wherein the medium comprises an adenosine receptor agonist.

13'. The method of any one of items 10'-12', wherein the ROCK inhibitor is Y27632 and the TGF-β type I receptor inhibitor is SB431542.

14'. The method of any one of items 10'-13', wherein the adenosine receptor agonist is NECA.

15'. The method of any one of items 10'-14', wherein the cancer tissue is a tissue obtained from a primary lesion.

16'. A method of treating colorectal cancer, comprising administering to a colorectal cancer patient in need thereof an effective amount of an FGFR inhibitor.

17'. The method of item 16', wherein the colorectal cancer patient is a patient selected by the method of any one of items 1'-15'.

18' The method of item 16' or 17', wherein the method further comprises selecting the colorectal cancer patient by the method of any one of items 1'-15'.

19'. The method of any one of items 16'-18', wherein the FGFR inhibitor is selected from erdafitinib, rogoratinib, AZD4547, BGJ398, BLU554, LY2874455, ASP5878, and ARQ087.

20'. The method of any one of items 16'-19', wherein the FGFR inhibitor is erdafitinib.

21'. The method of any one of items 16'-20', wherein the method further comprises administering an EGFR inhibitor.

22' The method of item 21', wherein the EGFR inhibitor is cetuximab.

23'. A pharmaceutical composition for treating colorectal cancer comprising an FGFR inhibitor.

24'. The pharmaceutical composition of item 23', wherein the pharmaceutical composition is administered to a colorectal cancer patient selected by the method of any one of items 1'-15'.

25'. The pharmaceutical composition of item 23' or 24', wherein the FGFR inhibitor is selected from erdafitinib, rogoratinib, AZD4547, BGJ398, BLU554, LY2874455, ASP5878, and ARQ087.

26'. The pharmaceutical composition of any one of items 23'-25', wherein the FGFR inhibitor is erdafitinib.

27'. The pharmaceutical composition of any one of items 23'-26', wherein the pharmaceutical composition is used in combination with an EGFR inhibitor.

28' The pharmaceutical composition of item 27', wherein the EGFR inhibitor is cetuximab.

29'. An FGFR inhibitor for use in treating colorectal cancer.

30'. The FGFR inhibitor of item 29', wherein the FGFR inhibitor is administered to a colorectal cancer patient selected by the method of any one of items 1'-15'.

31'. The FGFR inhibitor of item 29' or 30', wherein the FGFR inhibitor is selected from erdafitinib, rogoratinib, AZD4547, BGJ398, BLU554, LY2874455, ASP5878, and ARQ087.

32'. The FGFR inhibitor of any one of items 29'-31', wherein the FGFR inhibitor is erdafitinib.

33'. The FGFR inhibitor of any one of items 29'-32', wherein the FGFR inhibitor is used in combination with an EGFR inhibitor.

34'. The FGFR inhibitor of item 33', wherein the EGFR inhibitor is cetuximab.

35'. Use of an FGFR inhibitor for the manufacture of a medicament for treating colorectal cancer.

36'. The use of item 35', wherein the medicament is administered to a colorectal cancer patient selected by the method of any one of items 1'-15'.

37'. The use of item 35' or 36', wherein the FGFR inhibitor is selected from erdafitinib, rogoratinib, AZD4547, BGJ398, BLU554, LY2874455, ASP5878, and ARQ087.

38'. The use of any one of items 35'-37', wherein the FGFR inhibitor is erdafitinib.

39'. The use of any one of items 35'-38', wherein the FGFR inhibitor is used in combination with an EGFR inhibitor.

40'. The use of item 39', wherein the EGFR inhibitor is cetuximab.

The present invention is described in further detail in the following examples, which are not in any way intended to limit the scope of the invention.

Examples

Example 1: Preparation of spheroids of patient-derived tumor initiating cells (PD-TICs)

1. Methods

Spheroid culture of tumor initiating cells of colorectal cancer (CRC-TICs) and normal colonic epithelial cells

From each surgical specimen collected from 141 colorectal cancer (CRC) patients, 2 pieces of tumor (0.5-4 $cm^3$ each) and one piece of normal mucosa (~4 $cm^2$) were isolated, and stored in the ice-cold washing medium [DMEM/F12 with HEPES and 1-glutamine (Nacalai), 100 units/ml penicillin (Nacalai), 0.1 mg/ml streptomycin (Nacalai), and 10% bovine calf serum (SAFC Biosciences)]. Tissues were processed within 24 h after surgery. A piece of the CRC specimen (0.5-1.0 $cm^3$) or a flap of the normal mucosa (1-2 $cm^2$) was separated from the connective tissue, washed with PBS, and minced using scissors in a 60-mm petri dish. The tissue fragments were digested in 2 ml of collagenase solution [the washing medium supplemented with 0.2% collagenase type I (Thermo Fisher) and 50 $\mu$g/ml gentamicin (Thermo Fisher)] for 40-60 min at 37°C, and dissociated by pipetting with a 1 ml-pipette with ~20 min intervals 2-3 times. Then, cells were filtered through a 100-$\mu$m cell strainer (Corning) and collected. The cells were then resuspended in Matrigel (Corning), and placed in the center of each well of the 12-well cell-culture plate (~$1 \times 10^4$ - $5 \times 10^4$ cells/30 $\mu$l per well) . After polymerization of Matrigel at 37°C, the cells derived from CRC specimens were cultured with cancer medium [Advanced DMEM/F-12 (Thermo Fisher), 100 units/ml penicillin, 0.1 mg/ml streptomycin, 2 mM 1-glutamine (Nacalai), 10 $\mu$M Y27632 (R&D), 10 or 1 $\mu$M SB431542 (R&D), 5 $\mu$g/ml Plasmocin (Invivogen), and 5% FBS (Thermo Fisher)], and the cells derived from normal mucosa were cultured in eL-WRN medium [Advanced DMEM/F-12, 100 units/ml penicillin, 0.1 mg/ml streptomycin, 2 mM 1-glutamine, 50% L-WRL conditioned medium, 10 $\mu$M Y27632, 10 or 1 $\mu$M SB431542, 50 ng/ml EGF (Peprotech), 5 $\mu$g/ml Plasmocin, and 20% FBS]. The L-WRN CM, which is a conditioned medium of L-WRN cells (College of American Pathologists Consensus Statement 1999. Arch Pathol Lab Med. 2000; 124: 979-94.) was prepared according to a previous report (Gene. 1991; 108: 193-9). In some experiments, to the cancer medium was supplemented with 100 ng/ml bFGF (Peprotech), 50 ng/ml EGF, 1 $\mu$M NECA (Sigma), and/or B27 supplement (Thermo Fisher). A spheroid line was considered as successfully established when cancer spheroid culture was expanded to one plate (12 wells in a 12-well plate). At the end of the culture, the number of cells was ~$5 \times 10^4$ - $2 \times 10^5$ cells/well. The cancer spheroids thus obtained were stored in a freezer until use in each experiment. In the following experiments, the frozen cancer spheroid was thawed and passaged 5-20 times, and trypsinized to prepare cell aggregates each containing a few or few dozen of cells. The cell aggregates were then embedded in Matrigel (Corning) for use in experiments. The established spheroid lines were cultured with the minimum supplements.

Preparation of DNA and histology specimens from spheroids

Spheroids in Matrigel were suspended in Cell Recovery Solution (Corning), and collected in 1.5 ml-tubes. Matrigel was digested at 4°C with rotation for 30-60 min. Spheroids were centrifuged at 200 $\times$ g for 5 min, and washed with PBS twice at 4°C. DNA was purified using DNeasy Blood & Tissue Kit (Qiagen). For histological analyses, spheroids were passaged once or twice without trypsinization so that larger cell aggregates were formed. The spheroids thus obtained were embedded in iPGell (Genostaff), and fixed with 4% paraformaldehyde in PBS for 16 h at 4°C.

Histopathological classification of CRC

Formalin-fixed paraffin-embedded specimens were sectioned at 4 μm thickness, and stained with H&E. Histological grades of primary cancer and spheroids were determined according to the WHO guideline and the recommendation of the College of American Pathologists.

Mutational hotspot analysis

Hotspot mutations in 50 cancer-related genes were detected by Macrogen using Ion AmpliSeq Cancer Hotspot Panel v2 (Thermo Fisher) according to our order. The list of sequenced genes and mutations was available from manufacturer's website (http://tools.invitrogen.com/downloads/cms_106003.csv). The data sets were analyzed using Integrative Genomics Viewer software (Broad Institute).

Array-based comparative genomic hybridization (CGH) analysis

Array-based CGH analyses was performed with Agilent SurePrint G3 human CGH microarray 1x1M (G4447A, Agilent, Hachioji, Japan) and Genomic DNA ULS Labeling Kit (#5190-0419, Agilent) according to the manufacturer's instructions. Human genomic DNAs (G1521 and G1471, Promega, Madison, WI, USA) were used as references and controls. The array slides were scanned using Agilent G2565BA microarray scanner (Agilent).

Lentivirus infection of spheroids

A cDNA fragment encoding a firefly luciferase (luc2, Promega) was inserted into the pCX vector that contained a CAG promoter to construct pCX-Luc2. The expression unit (CAG-luciferase) of pCX-Luc2 was inserted into a lentiviral vector (pCDH, System Biosciences) to construct pCDH-CAG-Luc2. Lentiviral particles were prepared according to a protocol in a previous report (Gene. 1991; 108: 193-9) with minor modifications. Briefly, $2 \times 10^7$ of 293FT cells (Thermo Fisher) were seeded on four 10 cm-dishes, and cultured overnight. Cells were transfected with DNA of 40 μg pCDH-CAG-Luc2 plasmid, 26 μg psPAX2 packaging plasmid, and 14 μg pMD2.G envelope plasmid, with the use of Lipofectamine 2000 (Thermo Fisher) . Viral particles were harvested on the second and third days after transfection, and precipitated using PEG-it Virus-Precipitation Solution (System Biosciences). Viral particles were resuspended in 2 ml of the washing medium, aliquoted into 1.5 ml-tubes (50 μl each), and stored at -80°C. Spheroids cultured in one well of a 12-well cell-culture plate were trypsinized, and about half of the cells were collected by centrifugation (200 × g) in a 1.5 ml-tube. Cells were resuspended in 50 μl of the virus solution containing 10 μg/ml hexadimethrine bromide and 10 μM Y27632, and incubated for 6 h at 37°C. Then, the cells were collected by centrifugation (200 × g), resuspended in 60 μl of Matrigel, and seeded into two wells of a 12-well cell-culture plate.

Luminescence-based growth monitoring of spheroids

For growth monitoring assays, luciferase-expressing spheroids were cultured in 2-3 wells of a 12-well cell-culture plate. Spheroids were trypsinized, filtered through a 40-μm cell strainer (Corning), and diluted with the washing medium 4-8 times (based on the volume of Matrigel) to provide a concentration of $5 \times 10^4$ - $2 \times 10^6$ cells/ml depending on the growth rate and spheroid density. The spheroid cells were resuspended in Matrigel, distributed to the wells of 96-well "white" cell-culture plates (Corning; 3 μl per well) on a plate heater at 37°C, and cultured overnight in 100 μl/well of the medium. Spheroids were rinsed with 100 μl/well of PBS, and incubated with 50 μl of 150 μg/ml luciferin in phenol red-free DMEM/F12 medium (Nacalai) for 10 min at room temperature (20-2°C). Luminescence was scored using a conventional imaging device (Gel Doc XR+, BioRad). After this initial measurement, spheroids in each well were rinsed with 100 μl of PBS, and cultured in 100 μl of the medium. Luminescence was determined daily or 3 days later. The cell growth rate for each well was estimated as the proportion of photon counts to those on day 1. Four or six replicates (with similar statistical power) were analyzed for each data point in all experiments except for the chemical compound screening assays.

2. Results

Preparation of patient-derived colorectal cancer spheroids

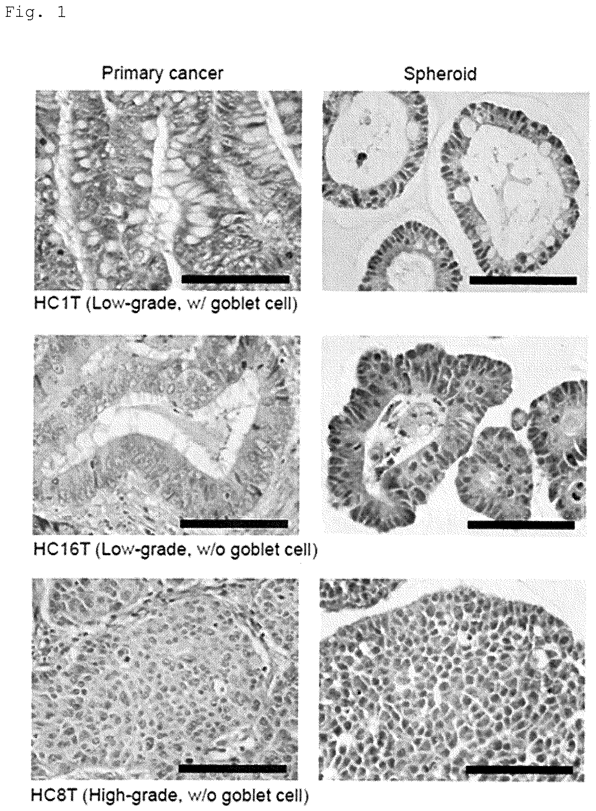

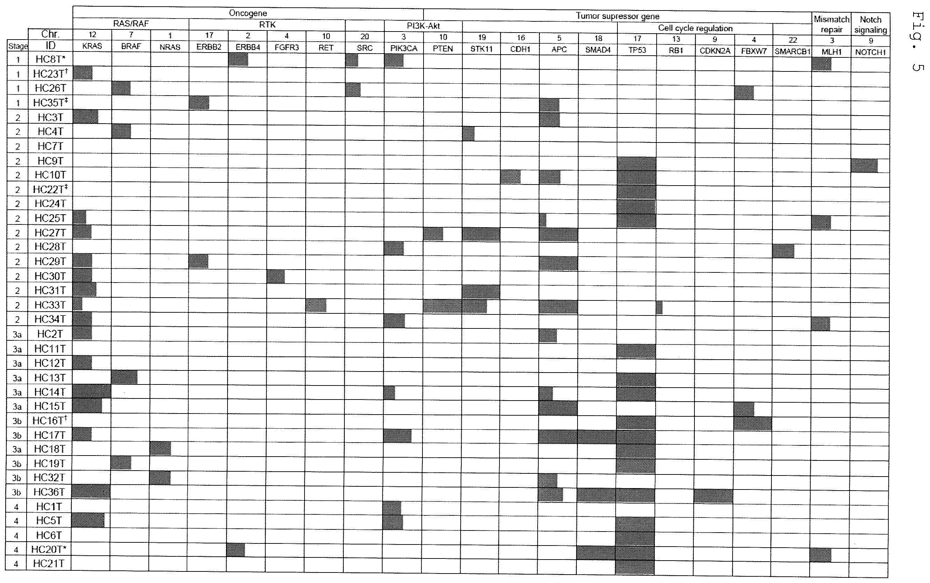

To culture patient-derived tumor initiating cell spheroids of colorectal cancer (PD-CRC-TIC spheroids), we prepared the "cancer medium" in which the basal medium (Advanced DMEM/F12) was supplemented with FBS (5%) and a Rock inhibitor Y27632 and a TGF-β type I receptor inhibitor SB431542. To culture spheroids of normal colonic epithelial cells from the same patients, we also prepared "the eL-WRN medium" by supplementing the L-WRN conditioned medium with Y27632 and SB431542. We cultured 148 colorectal cancer samples from 141 colorectal cancer patients. Pathologically, the samples were classified as low-grade (138/148), high-grade (5/148), and mucinous (5/148) adenocarcinomas according to the standard histological criteria. The histological features of the cancer epithelium coincided well between the primary tumors and their spheroids (Fig. 1). To characterize the key genetic mutations, we sequenced 50 cancer-related genes of 36 CRC-TIC spheroid lines (Fig. 5) .

Luciferase-based growth monitoring of spheroids

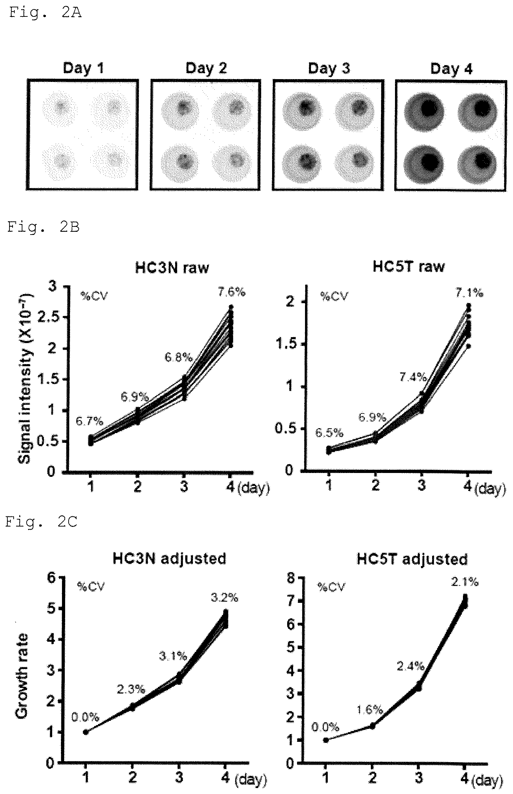

It was difficult to determine the number of live cells in 3D culture and the growth rate of the cells. To determine the number of cells in spheroid culture precisely, we utilized spheroids infected with a lentivirus containing a luciferase expression vector driven by CAG promoter. Because high efficiency of infection eliminated the necessity to enrich expressor subpopulations, we passaged post-infection spheroids 2-3 times without subcloning, and directly subjected them to cell growth assays. We monitored the growth of the normal and cancer spheroid lines daily from post-passage days 1 to 4 (Fig. 2A). As shown in Fig. 2B, bioluminescence levels among 16 replicated wells varied rather widely, reflecting differences in the initial cell numbers (coefficient of variation, 6.5-7.6%). However, when calibrated to the initial photon counts on day 1, the variations were reduced to 1.6-3.2% (Fig. 2C). Thus, the present method was a reliable method to monitor spheroid growth.

Stimulation of CRC-TIC spheroid growth with EGF and bFGF

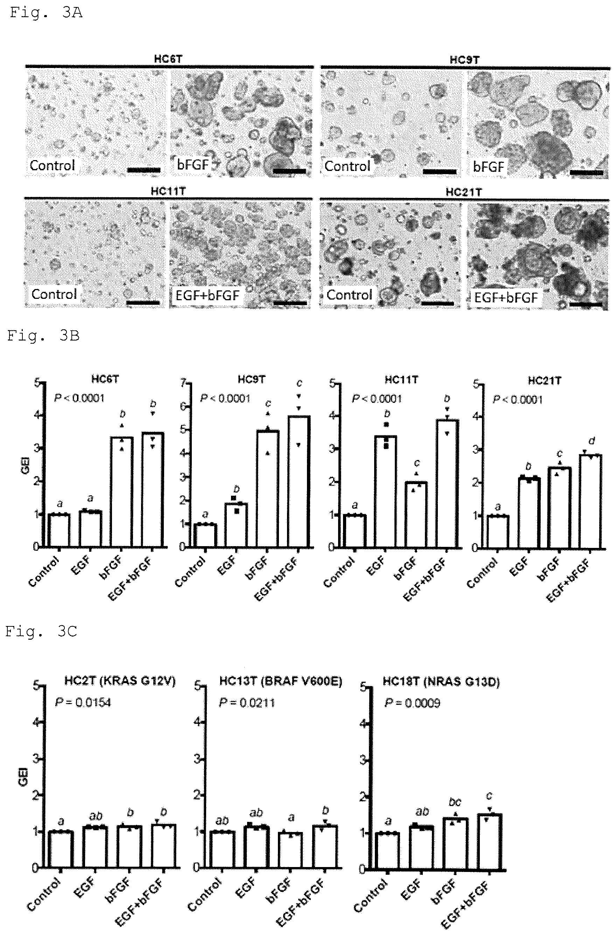

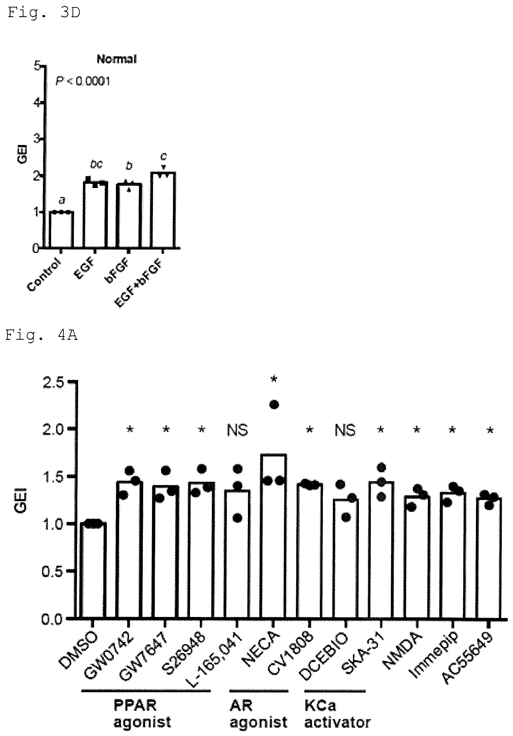

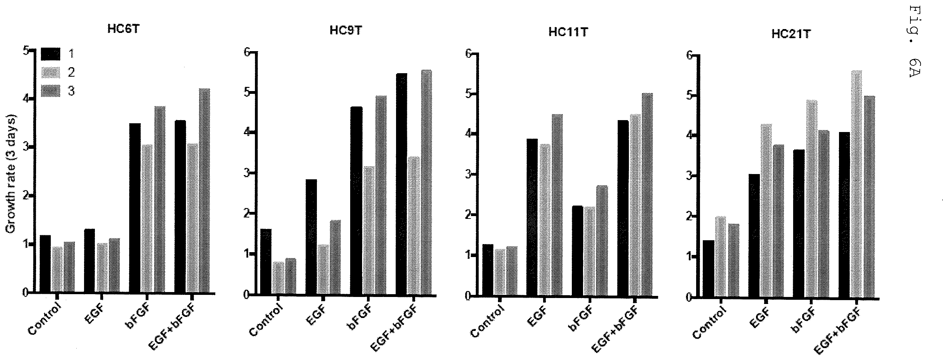

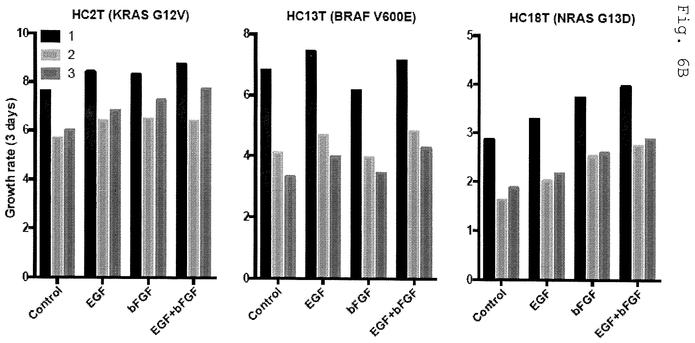

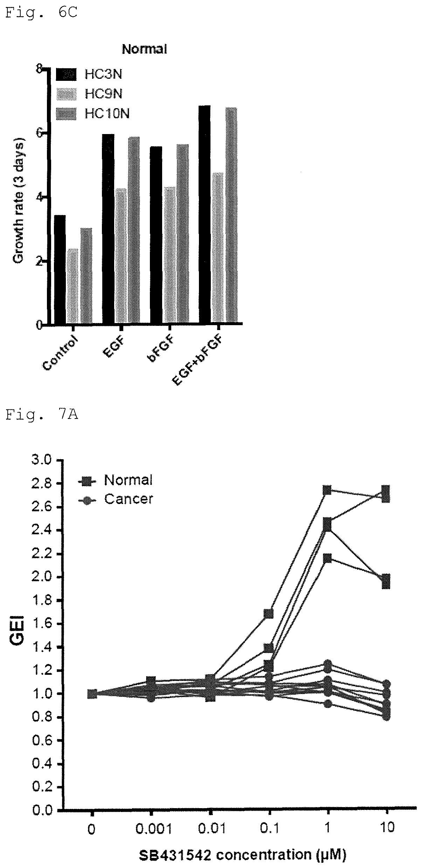

To investigate whether EGF and bFGF helped spheroid proliferation in the cancer medium as described above, we cultured with EGF and/or bFGF four slow-growing CRC-TIC spheroid lines that carried wild-type *RAS*/*RAF* genes (HC6T, HC9T, HC11T, HC21T). Because EGF/bFGF facilitated the growths of these spheroid lines substantially (Fig. 3A), we then quantified their effects by the luminescence-based growth monitoring. To calibrate the effects, we introduced the growth effect index (GEI) defined as the relative growth rate of a treated group to that of its solvent-only control in each paired assay. We found that additions of both EGF and bFGF more than doubled the GEI in all four spheroid lines that carried the wild-type *RAS*/*RAF* genes (Fig. 3B; and Fig. 6A). The extents of growth promotion by EGF and bFGF were different depending on the spheroid line. In two lines (HC6T, HC9T), bFGF was more effective than EGF, whereas in one line (HC11T), vice versa (Fig. 3B). In another spheroid line (HC21T), the effects were similar between the two growth factors (Fig. 3B). EGF/bFGF showed only limited growth-promoting effects on three spheroid lines that carried RAS or RAF mutations (HC2T, HC13T, HC18T) (< 55% increases by both; Fig. 3C; and Fig. 6B). EGF and bFGF doubled the growth of normal colonic epithelial cell spheroids, too (Fig. 3D). Two spheroid lines HC6T and HC9T showed gain of the FGF9 chromosomal region, which may explain better GEI of bFGF in these lines compared with those in normal spheroids.

Identification of growth-promoting supplements through chemical library screening

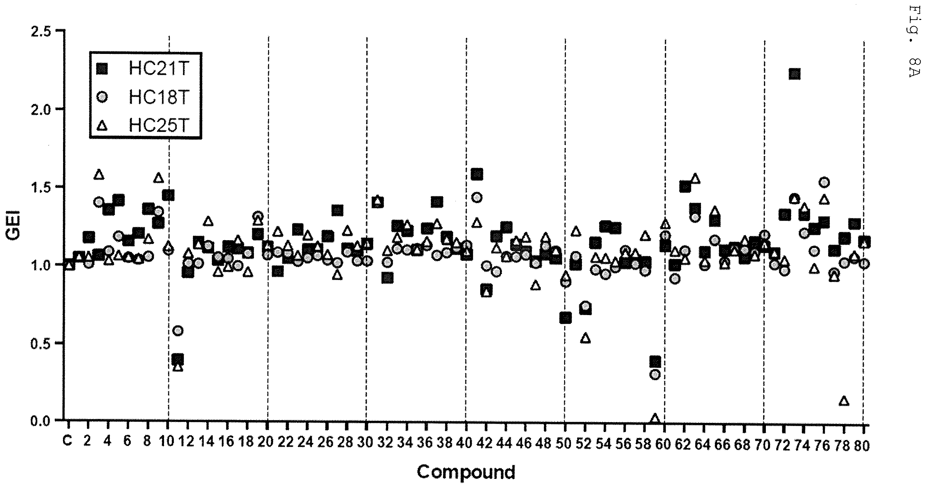

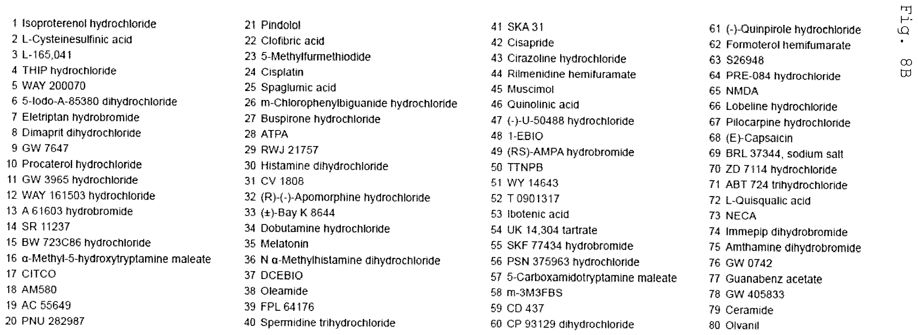

To find additional supplementary factors that could promote spheroid growths, we screened 80 pharmacologic agonists or activators for growth-promoting effects on three luciferase-expressing CRC-TIC spheroid lines with moderate growth rates (i.e., 3-4 times in 3 days) (HC21T, HC18T, HC25T). We identified eleven compounds that stimulated spheroid growths by > 25% (Fig. 4A; Fig. 8A and 8B). The 11 compounds included two adenosine receptor (AR) agonists (NECA, CV1808), four PPAR agonists (GW7647, GW0742, S26948, L-165,041), and two calcium-activated potassium-channel (KCa) activators (DCEBIO, SKA-31), one NMDA-type glutamate receptor agonist (NMDA), one histamine H3 receptor agonist (imepip), and one retinoic acid receptor (RXR) $\beta$2 agonist (AC55649) (10 $\mu$M each).

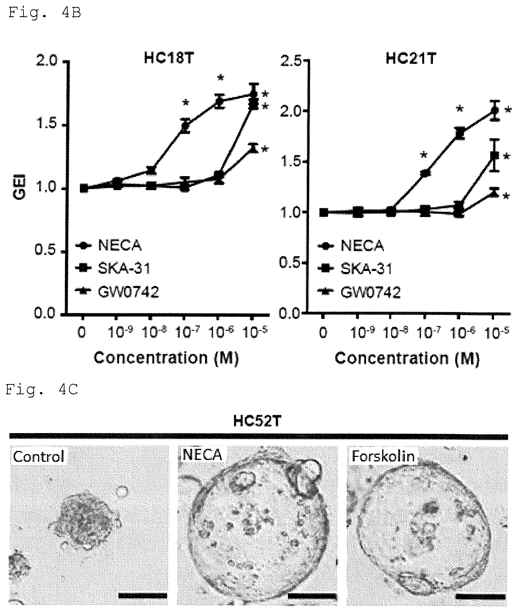

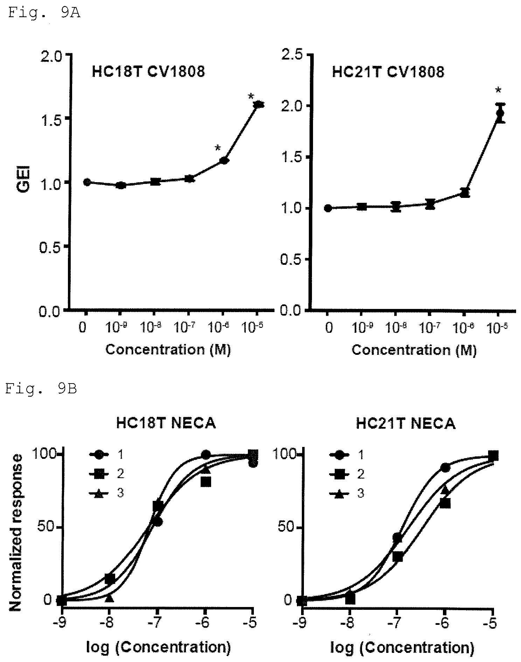

We then performed titration experiments for GW0742 (PPAR$\delta$ agonist), NECA (non-selective AR agonist), and SKA-31 (activator of KCa2 and KCa3.1a). While NECA exhibited a strong activity of growth promotion ($EC_{50}$ = 63.1 and 195 nM in HC18T and HC21T spheroid lines, respectively), other two compounds showed significant effects at 10 $\mu$M (Fig. 4B). We also confirmed that another AR agonist, CV1808 (2-phenylaminoadenosine), showed a growth-promoting effect though weaker than that of NECA (Fig. 9A). These results suggest that AR agonists, in particular NECA, are suitable for CRC-TIC spheroid cultures. Dose-dependent responses for NECA in three independent experiments were highly reproducible (Fig. 9B), underscoring the reliability of our spheroid culture in drug-sensitivity tests.

Stimulation of CRC-TIC spheroid growth with NECA through cAMP-PKA signaling pathway

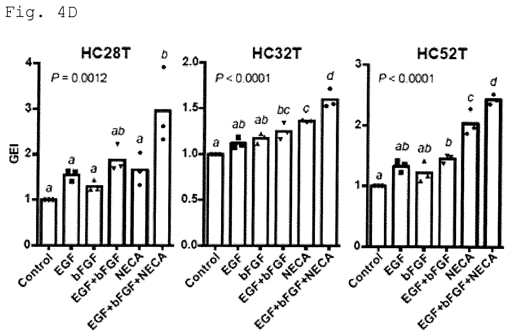

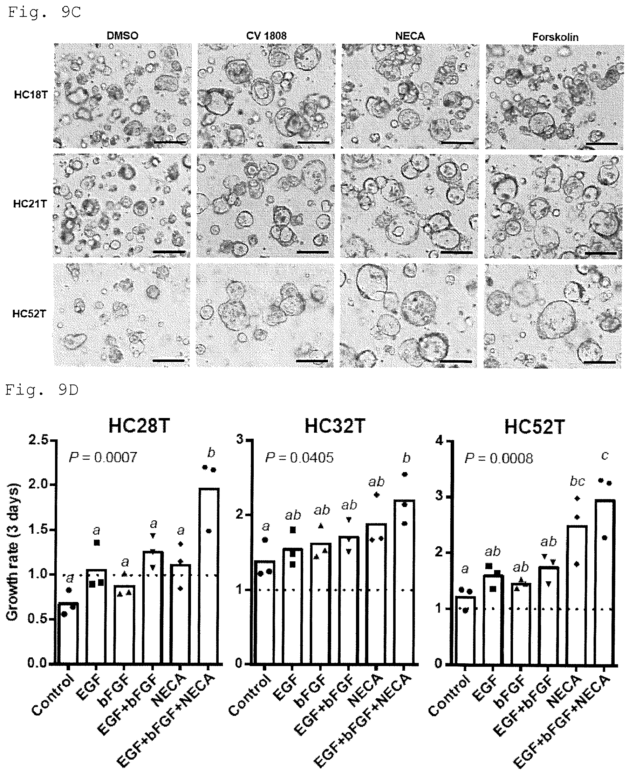

Because $A_{2B}$ is the only the adenosine receptor expressed in the colonic epithelium, NECA appears to stimulate the cyclic AMP (cAMP)-protein kinase A (PKA) signaling in these cells. Consistently, NECA as well as CV1808 induced morphological changes in spheroids characterized by cell flattening and increased sphere size, similar to those in forskolin-treated spheroids (Fig. 4C; and Fig. 9C). Supplementation with NECA in addition to EGF and bFGF enabled us to stably maintain three slow-growing spheroid lines (HC28T, HC32T, HC52T), for which EGF and bFGF alone were ineffective in growth stimulation. Accordingly, we infected these spheroid lines with a lentiviruses and make them express a luciferase gene to monitor their growth. By the luciferase assay, we confirmed that addition of EGF, bFGF and NECA significantly stimulated their growths by 50% or more (Fig. 4D). These spheroid lines barely grew in the cancer medium alone (growth rates, ~1.0) (Fig. 9D). These results suggest that activation of the cAMP-PKA and MAPK/ERK signaling enhances proliferation of CRC-TIC spheroids. Thus, we can cultivate even some extremely slow-growing spheroids by supplementing the basal cancer medium with three additional ingredients, EGF, bFGF, and NECA.

Optimized culture conditions for PD-TIC spheroids

On the basis of the above results, we modified culture media for both normal and cancer SC spheroids. In the periods A-D in Table 1, EGF and bFGF were added to the standard medium only when the growth with the medium was insufficient. We optimized its concentration of SB431542 in the period V to 1 μM to avoid its growth inhibition often seen at 10 μM (Fig. 7A). As B27 supplement (Thermo Fisher) promoted CRC-TIC spheroid growth (Fig. 7B), we included EGF, bFGF, NECA and B27 supplement (Thermo Fisher) in the initial culture of PD-CRC-TICs in the period E in Table 1.
The overall success rate for establishing CRC-TIC spheroids was 73% (108/148). The reasons for unsuccessful CRC-TIC spheroid culture included contamination by microorganisms (bacteria or yeasts; 4 cases), cell damage by radiation therapy before surgery (2 cases), and poor cell growth followed by cell death in early passages (of unknown causes; 34 cases). We found no statistical correlations between the success rates and tumor stages (Table 2). As a result of the optimizations of culture conditions, the success rate for establishing CRC-TIC spheroids increased to ~90% in the period E in Table 1 (Table 1).

Table 1

Success rates for establishing CRC-TIC spheroids

| Period | A<br>11/13/2014 -<br>6/30/2015 | B<br>7/1/2015 -<br>12/31/2015 | C<br>1/1/2016 -<br>6/30/2016 | D<br>7/1/2016 -<br>12/31/2016 | E<br>1/1/2017 -<br>3/31/2017 |
|---|---|---|---|---|---|
| Total attempts | 22 | 45 | 34 | 23 | 24 |
| *Success (%) | 14 (64%) | 31 (69%) | 26 (76%) | 16 (70%) | 21 (88%) |
| Numbers of CRC samples from which spheroid lines were successfully established. | | | | | |

Table 2

Tumor stages of CRC samples

| Stage | I | II | III | IV | Total |
|---|---|---|---|---|---|
| Number | 17 | 64 | 48 | 19 | 148 |
| *Success (%) | 15 (88%) | 42 (66%) | 37 (77%) | 14 (74%) | 108 (73%) |
| * Numbers of CRC samples from which spheroid lines were successfully established. | | | | | |

Reference Example 1: Drug sensitivity tests in PDSX models (1) Comparison between PDX model and PDSX model

To compare PDX model and PDSX model in application with drug sensitivity tests, we established PDX and PDSX models from 92 samples obtained from 89 colorectal cancer patients who underwent surgical resections.
Tumor samples were washed with the washing medium (DMEM/F12 containing 10% bovine calf serum, 100 U/ml penicillin, and 0.1 mg/ml streptomycin) and PBS twice each. Necrotic tissues of the tumor samples were removed as much as possible. After washing, we divided the tumor samples into cubic fragments of 50-100 mm$^3$ and used to establish cancer spheroids and PDXs.
To establish $P_0$ (founder) PDXs, the tumor fragments were implanted directly to each flank of nude mice and/or NOD-SCID mice with under isoflurane anesthesia. The growth of implanted tumors was measured with a pair of calipers at least once a week, and the volume of each tumor was estimated using the following formula; tumor volume (mm$^3$) =

[length (mm) $\times$ width$^2$ (mm$^2$)] / 2 (the length means the longer tumor axis and the width means the shorter tumor axis). Implantations were judged as successful when estimated tumor volumes reached ~1000 mm$^3$, and as failed if no visible tumor masses were recognized for 6 months after transplantation. To passage the tumors, we excised them and divided them into cubic fragments of 50-100 mm$^3$. These fragments were transplanted into nude mice to establish the next generation of PDXs.

Patient-derived spheroids used in this example were obtained by thawing the frozen samples of the 21 lines established in Example 1 using the cancer medium, which is a basic medium (Advanced DMEM/F-12) supplemented with FBS (5%), a ROCK inhibitor (Y27632) and a TGF-$\beta$ type I receptor inhibitor (SB431542), or the cancer medium further comprising EGF and bFGF. The spheroids were cultured with the medium used when they were established.

PD-CRC-TIC spheroids were cultured in a 12-well culture plate. Spheroids in confluent culture ($4 \times 10^5$ - $4 \times 10^6$ cells/well) were rinsed with PBS twice, and transferred to a 1.5-ml tube together with Matrigel. The volume of the spheroid suspension was adjusted to 100 $\mu$l with PBS, and the suspension thus obtained was injected into nude mice subcutaneously at $1 \times 10^5$ - $9 \times 10^5$ cells/mouse.

We successfully established P$_0$ (founder) generation PDX mice in 56 of 92 cases (61%), and PD-CRC-TIC spheroids in 68 of 92 cases (74%).

To determine the efficiency in establishing xenograft models from the PD-CRC-TIC spheroids, we injected nude mice with 21 PD-CRC-TIC spheroid lines subcutaneously, and successfully established model mice with 18 lines (86%). In contrast, the success rate for passaging from the P$_0$ (founder) to P$_1$ generation PDXs was 70%. For drug-dosing tests, a large number of mice enough for a strong statistical power (e.g., n = 3-6 for each group) has to be prepared. Accordingly, for PDX models, serial transplantations are inevitable with two or more passages. On the other hand, for PDSX models, we can generate enough numbers of PDSX mice simultaneously once we propagate spheroids *in vitro*.

In 56 cases that generated P$_0$ (founder) PDXs, 148 of 236 (63%) samples formed P$_1$ PDXs. In contrast, 198/228 (87%) of spheroid-injected mice developed PDSX mice. PDSX models showed much smaller variances in tumor volumes among individual mice than PDX models. The coefficients of variation (CVs) for P$_1$ PDXs and PDSXs were 0.53 and 0.29, respectively.

To evaluate the reliability in drug sensitivity tests with PDX and PDSX models, we prepared test mice transplanted with samples from 4 colorectal cancer patients. We had to exclude 23 of 94 (24%) PDXs as outliers because of the large individual variances in growth rates. In contrast, we eliminated 9 of 85 (11%) PDSXs according to the same criteria.

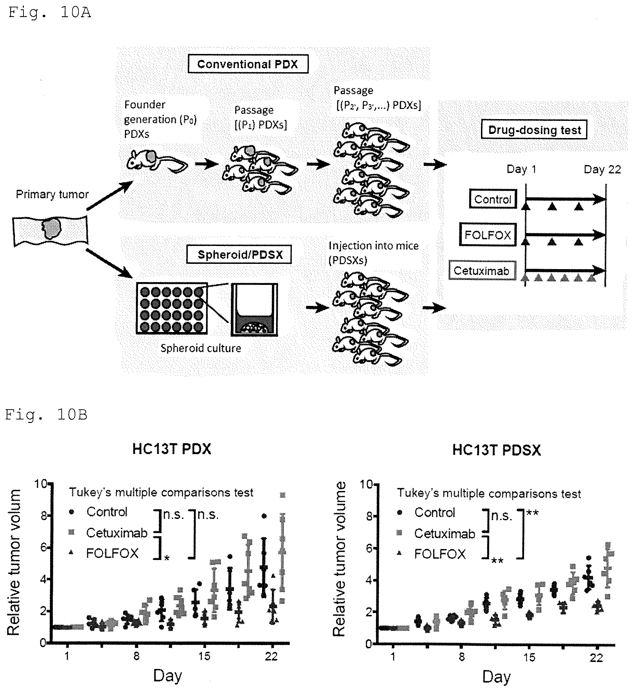

First, we prepared PDX and PDSX models derived from a CRC case (HC13T) that carried a *BRAE*$^{V600E}$ mutation, and performed a drug sensitivity test (Fig. 10A).

PDX (P$_2$-P$_4$) and PDSX mice were prepared and subjected to the drug sensitivity test when the estimated tumor volumes reached 300-500 mm$^3$. Mice were distributed into the control and treatment groups (n = 3-6 per group). The day of the dosing start was defined as day 1, and the tumor volume and the body weight of each mouse were measured twice a week for 3 weeks (day 1-22). The treatment protocols were designed to reflect clinical regimens and drug doses for mice were calculated according to the following formula: mouse dose (mg/kg) = human dose (mg/kg) $\times$ 37 (human $K_m$)/ 3 (mouse $K_m$). For the FOLFOX-like treatment, mice were injected intraperitoneally (i.p.) with oxaliplatin (12 mg/kg) and levofolinate calcium (30 mg/kg) first, followed by 5-fluorouracil (55 mg/kg), weekly for 3 weeks. For cetuximab treatment, mice were injected intraperitoneally (i.p.) at 250 $\mu$g per injection, twice a week for 3 weeks. These doses were less than the maximal tolerated doses and approximately 80% of the clinically relevant doses. Relative tumor volumes were calibrated to the initial tumor volumes on day 1. To evaluate effects of treated drugs, T/C (Treated/Control) and TGI (Tumor Growth Inhibition) values were calculated. The T/C was defined as the ratio of relative tumor volume in a treated group to that in the untreated group, which indicates tumor growth inhibition by a treatment. The value of T/C was calculated by the following formula. The value of TGI was also calculated by the following formula and used to assess tumor growth inhibition.

```
T/C (%) = relative tumor volume (treated) / relative tumor
volume (control) × 100.

TGI (%) = [1 - Δrelative tumor volume (control)/ Δrelative
tumor volume (treated)] × 100,
```

or

$$\text{TGI (\%)} = [1 - \Delta\text{relative tumor volume (control)}] \times 100 \text{ for}$$
$$\Delta\text{relative tumor volume (control)} < 0 \text{ (tumor regression)}.$$

$$\Delta\text{relative tumor volume} = (\text{relative tumor volume on day 22})$$
$$- (\text{relative tumor volume on day 1}).$$

Cetuximab was ineffective in both PDX and PDSX models (T/C; 120% and 114% with PDXs and PDSXs, respectively), the FOLFOX-like regimen significantly decreased the tumor growth (T/C; 48% and 57% with PDXs and PDSXs, respectively) (Fig. 10B). The data with PDSXs showed much smaller variances than that with PDXs at the corresponding data points (Fig. 10B). Essentially, the same results were obtained in three additional patient tumors. Namely, all data sets with PDSXs had only half as large CVs as those with PDXs [CV (mean): 15 and 29 for PDSXs and PDXs, respectively].

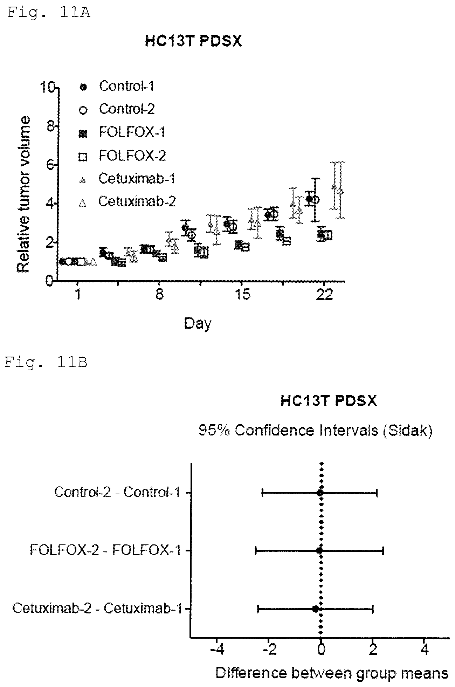

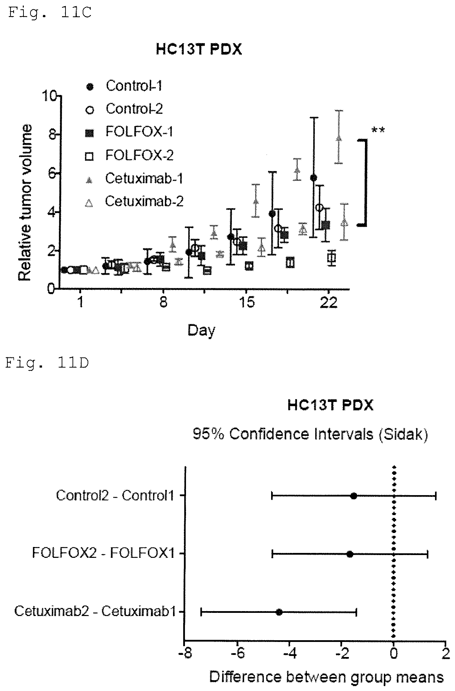

To investigate the reproducibility, we performed drug sensitivity tests with two sets of PDSXs (spheroids at 12th and 16th passages; $P_{12}$ and $P_{16}$) and PDXs ($P_2$ and $P_3$ generations) independently prepared from HC13T tumor. The relative tumor volumes of the two sets with PDSXs showed a stronger correlation [Spearman r = 0.99, 95%CI: 0.96-0.99] than those with PDXs [Spearman r = 0.87 95%CI: 0.69-0.95] (Figs. 11A-11D). We obtained similar results with additional two cases, underscoring high reproducibility of PDSX models. Thus, PDSX models enabled us to decrease individual variances in the growth rates and evaluate drug sensitivity even with such small numbers of mice as three to four in each group.

(2) Drug sensitivity in PDSX models that reflects clinical outcome of patients

We compared drug responses of PDSXs with the clinical outcome of the patients in a retrospective manner. We prepared panels of PDSXs from seven patients who had been treated with chemotherapies against metastases after resection of their primary tumors. We performed a total of nine drug-dosing tests that matched chemotherapeutic regimens in the corresponding patients. Notably, eight of the nine sets (89%) of the drug-dosing results with PDSXs well reflected their corresponding clinical outcomes. Namely, the therapeutic regimens that were efficacious in the patient [partial response (PR) or stable disease (SD) with RECIST] were also effective in treating PDSXs, whereas those that were not efficacious in the patients [progressive disease (PD) with RECIST] were ineffective in PDSXs. In addition, there was a strong correlation between the results of PDSX dosing tests (Treated/Control, T/C) and patient outcomes [the best response with RECIST (BR) (r = 0.70, p = 0.04) and the durations of clinical responses in patients (DOR) (r = -0.77, p = 0.04)]. A strong correlation was also observed between TGI (Tumor Growth Inhibition) and patient outcomes [both BR (r = -0.72, p = 0.03) and DOR (r = 0.83, p = 0.02)].

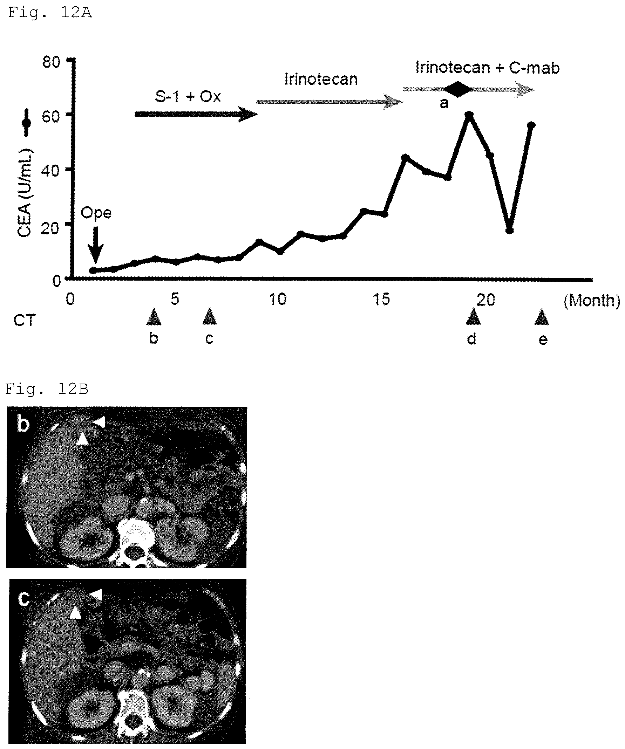

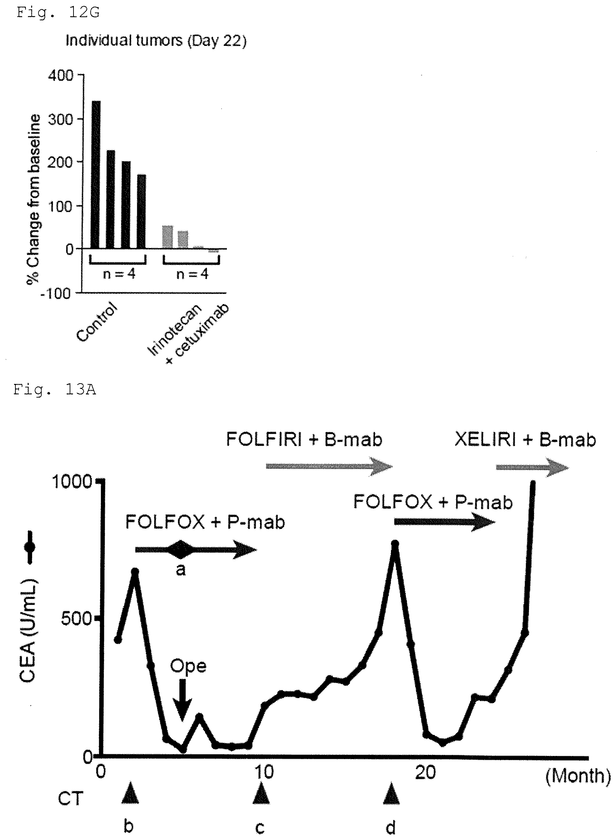

Results of representative patients are shown in Figs. 12A-12G and Figs. 13A-13D. For FOLFOX-like treatment to PDSX models, mice were injected intraperitoneally (i.p.) with oxaliplatin (12 mg/kg) and levofolinate calcium (30 mg/kg) first, followed by 5-fluorouracil (55 mg/kg), weekly for 3 weeks. For irinotecan treatment, mice were injected (i.p.) at 40 mg/kg weekly for 3 weeks. For FOLFIRI-like treatment, mice were injected (i.p.) with irinotecan (40 mg/kg) and levofolinate calcium (30 mg/kg) first, followed by 5-fluorouracil (55 mg/kg), weekly for 3 weeks. For cetuximab treatment, mice were injected (i.p.) at 250 $\mu$g per injection, twice a week for 3 weeks.

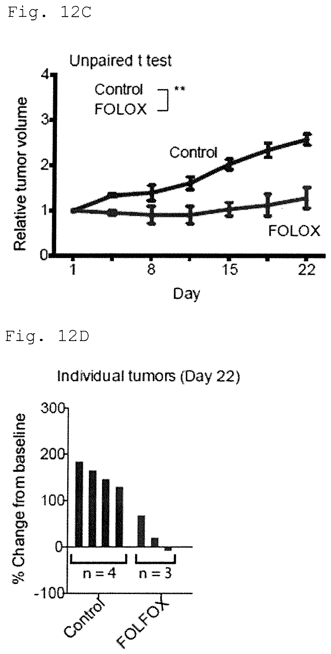

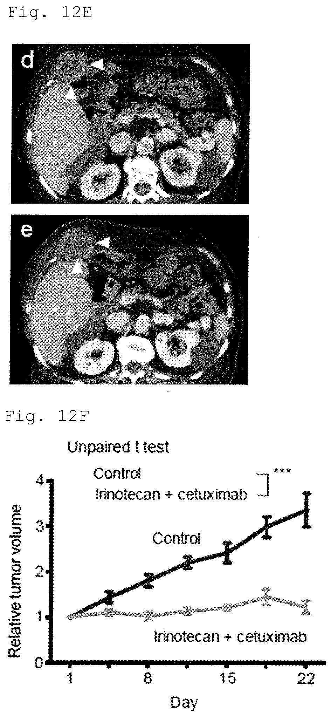

Patient 1 in Figs. 12A-12D suffered from a right side colon cancer with peritoneal metastasis, and underwent surgical resection of the primary lesion. Then, the patient was treated with S-1 (a 5-FU prodrug) + oxaliplatin (a FOLFOX-like regimen) that led to SD (Stable disease) for 4 months (Figs. 12A and 12B). Another FOLFOX-like treatment in PDSXs resulted in a moderate response (T/C = 50%, TGI = 82%) with a significant difference from the control group (Figs. 12C and 12D). Later, the patient was treated with irinotecan + cetuximab, which resulted in SD with the DOR of 6 months (Figs. 12A and 12E), although the treatment was interrupted for a month due to a knee bone fracture caused by an accident. The serum CEA level of this patient increased rapidly by the interruption accompanied with metastatic expansion. However, resumption of the treatment rapidly reduced the CEA level for 2 months (Fig. 12A). We also performed the same irinotecan + cetuximab regimen on PDSX mice, which caused a moderate response (T/C = 37%, TGI = 93%) (Figs. 12F and 12G) . Accordingly, both sets of these clinical results were reproduced well in the dosing experiments in the corresponding PDSXs.

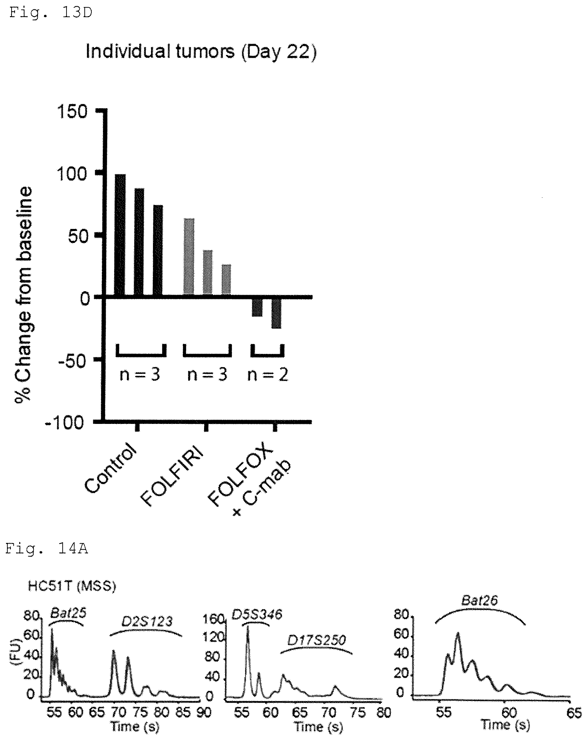

Patient 3 in Figs. 13A-13D suffered from a left side colon cancer with liver metastases. First, the patient was treated with FOLFOX + panitumumab (an anti-EGFR antibody) regimen that resulted in a dramatic decrease in the tumor volume and serum CEA level. After resection of the primary lesion, the patient received FOLFOX+ panitumumab again, which resulted in PR [DOR; 4 months (2nd FOLFOX + panitumumab treatment)] (Figs. 13A and 13B). Following 5 months of the 2nd FOLFOX + panitumumab treatment, the serum CEA level started to raise, suggesting the resistance to the regimen. Then the patient was switched to FOLFIRI + bevacizumab regimen that led to SD (DOR; 5 months) (Figs. 13A and 13B). In the PDSX models, the tumor was sensitive to FOLFOX + cetuximab, which caused PR (T/C = 42%, TGI = 122%) (Figs. 13C and 13D). On the other hand, the PDSX models did not respond to the FOLFIRI regimen (T/C = 76%, TGI = 50%) Fig. 13C). Accordingly, the ineffective results of FOLFIRI-based treatment appear to have been the innate characteristic of the primary cancer, which was reflected in PDSX data.

The PDX models have been reported to be predictive of clinical responses. To further evaluate the predictive power of PDSXs, we compared the results of drug dosing tests (T/C and TGI) on PDSXs with those on PDXs derived from the same patient tumors. The results of PDSXs showed a strong concordance with those of PDXs in both T/C [r = 0.93 (95%CI: 0.63 to 0.99), p = 0.001] and TGI [r = 0.93 (95%CI: 0.64 to 0.99), p < 0.001]. These results support the robust reliability of PDSXs in non-clinical evaluation of drug sensitivity.

One of the most practical limitations of PDXs for personalized medicine is the long time that is needed to prepare enough numbers of PDX mice for drug-dosing tests. For example, it took 5 months or longer to prepare $P_2$ generation PDXs in our study. [More precisely, it took 70 days (median) for the $P_0$ (founder) generation tumors to expand to 1,000 $mm^3$, and ~50 days for the $P_1$ generation to reach the same size]. Thereafter, it took ~ a month for $P_2$ PDXs to grow up to the size of 300-500 $mm^3$ (appropriate for drug dosing). In contrast, it took us for only 2-3 months to prepare a group of ~20 PDSXs mice. [~ a month to establish spheroids from the primary lesion followed by 1-2 more weeks to expand them to enough numbers of spheroids for injection into mice.] Thereafter, it took ~24 days to expand PDSXs to the size of 300-500 $mm^3$. These results indicate that the PDSX method is far more advantageous than the PDX method not only in the accuracy of drug sensitivity testing but also in saving time for preparation of the tumor-bearing mice. We can expect to feedback the dosing data to the bedside before deterioration of the patient conditions.

Reference Example 2: Detection of microsatellite instability (1) Detection of microsatellite instability (MSI) using spheroid-derived DNA samples and immunohistochemistry for mismatch repair (MMR) proteins in colorectal cancer

We established 111 cancer cell spheroid lines and paired 11 normal epithelial cell spheroid lines from surgical specimens of 110 colorectal cancer patients. One of the patients had double cancers.

The 94 cancer spheroids established in the examples with the cancer medium (a basic medium (Advanced DMEM/F12) supplemented with FBS (5%), a ROCK inhibitor (Y27632) and a TGF-β type I receptor inhibitor (SB431542)) with or without EGF and bFGF were cultured in the same medium as that used at the time of establishment. The spheroids of normal colon epithelial cells from the same patients were cultured in "eL-WRN medium", in which Y27632 and SB431542 were added to L-WRN conditioned medium. DNA was extracted from about $2 \times 10^5$ to about $1 \times 10^6$ cells from each spheroid.

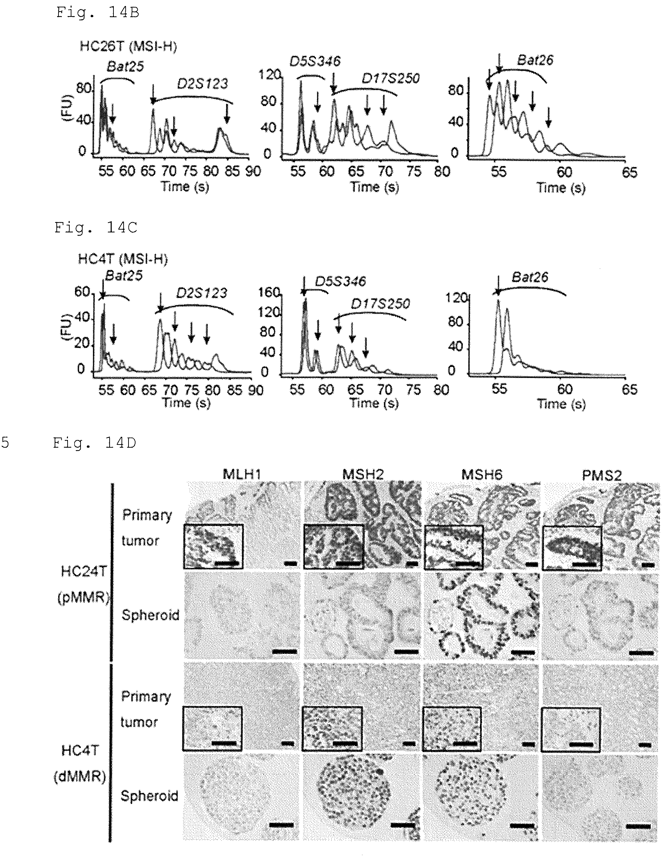

For all the 111 patients, we employed an on-chip MSI test that analyzed PCR products amplified from DNA extracted from cancer and normal spheroids on Agilent Bioanalyzer chips. The MSI status was determined using five microsatellite markers BAT25, BAT26, D5S346, D2S123, and D17S250 (Bethesda panel). In microsatellite stable (MSS) cases, the peak patterns of PCR products were concordant closely between cancer cells and paired normal cells (Fig. 14A), and a shifted peak was confirmed in MSI-positive cases (Figs. 14B and 14C). We called MSI-H when two or more showed shifted peaks among the five markers. If only one of the five markers showed a shifted peak, we called MSI-L, and then tested two more markers; BAT40 and MYCL. Using this analysis, we detected 8 (7.2%) MSI-H, 30 MSI-L, and 73 MSS cases.

Next, to confirm the correlation between MSI status and MMR deficiency, immunohistochemistry (IHC) of MMR proteins (MLH1, MSH2, MSH6 and PMS2) were performed in primary lesion of all 111 cases. As a result, 8 MMR deficiency cases and 87 MMR proficient cases were detected (Fig. 14D). One inconsistent case between results of MSI test and IHC were existed (HC13T). We also stained MMR proteins in formalin-fixed paraffin-embedded spheroid samples. Except one case (HC13T), the results of IHC between primary lesion and spheroids samples were accordant. Although HC13T was diagnosed as MMR proficient by the on-chip MSI test of spheroid samples and sequence analysis of MMR proteins, the analysis of primary lesion samples detected MLH1 and PMS2 were missing. This discrepancy was likely caused by the poor quality of the IHC analysis on the primary lesion as the IHC on the spheroids detected MLH1 and PMS2. From the results of the on-chip MSI test and IHC in both primary lesion and spheroid samples in 111 cases, 8 cases (7.2%) were determined as MSI-H (HC4T, HC8T, HC26T, HC49T, HC44T, HC106T, HC114T and HC137T).

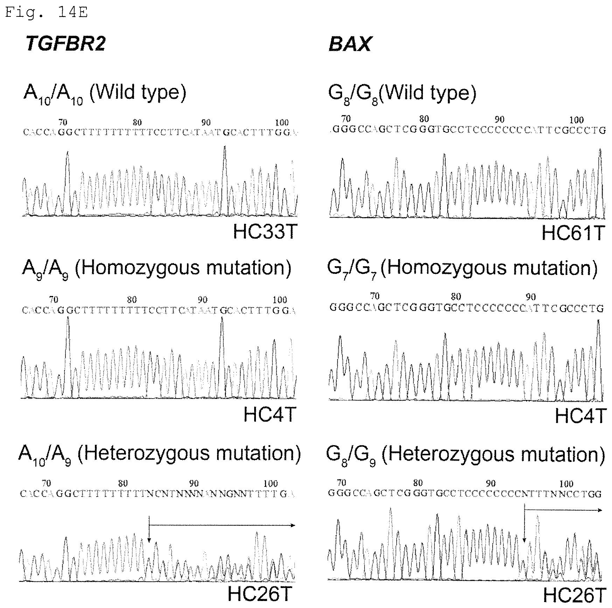

Next, we employed sequence analysis to determine mutations in mononucleotide repeats in TGFBR2, BAX, IGF2R and CAPSP5. The high purity of the spheroid-derived DNA samples allowed us to detect heterozygous mutations (Fig. 14E) .

(2) Accurate MSI analyses using spheroid-derived DNA

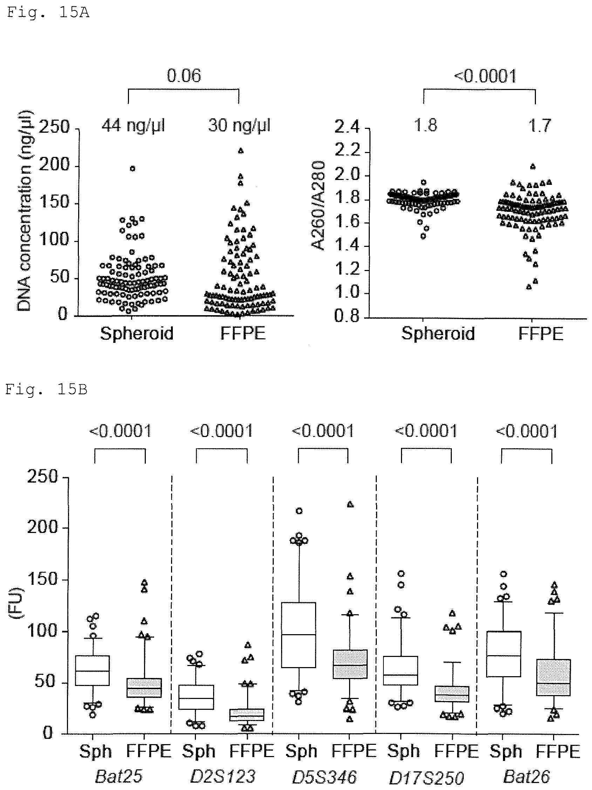

To compare the quality of spheroid-derived DNA samples to that of formalin-fixed and paraffin-embedded (FFPE) tissue-derived DNA samples, we performed the on-chip MSI test with FFPE tissue-derived DNA samples and spheroid-derived DNA samples in 52 cases. First, the quality of both DNA samples was checked. The median A260/A280 of spheroid-derived DNA samples was significantly higher than that of FFPE tissue-derived DNA samples (1.8 vs 1.7, p < 0.0001), and the median concentration of spheroid-derived DNA samples was also higher than that of FFPE tissue-derived samples (44 ng/$\mu$l vs 30 ng/$\mu$l, p = 0.06) (Fig. 15A). The MSI test using FFPE tissue-derived DNA samples detected 4 (8.0%) MSI-H cases and 46 MSI-L and MSS cases (MSI-L: 14 cases, MSS: 32 cases). On the other hand, the test using spheroid-derived DNA sample detected 5 cases (10%) as MSI-H and 45 cases as MSI-L and MSS.

For all loci tested, the peak heights of PCR products amplified from the spheroid-derived DNA were significantly higher than those of FFPE tissue-derived DNA (Fig. 15B). According to the results of the on-chip MST test, IHC of MMR proteins, and sequence analysis of MMR-deficiency target genes, the sensitivity and specificity of the MSI test with spheroid-derived DNA samples was both 100%, whereas those with the FFPE tissue-derived DNA was 60% and 98%, respectively. These results showed that spheroid derived-DNA sample analysis was more sensitive than FFPE tissue-derived DNA sample analysis.

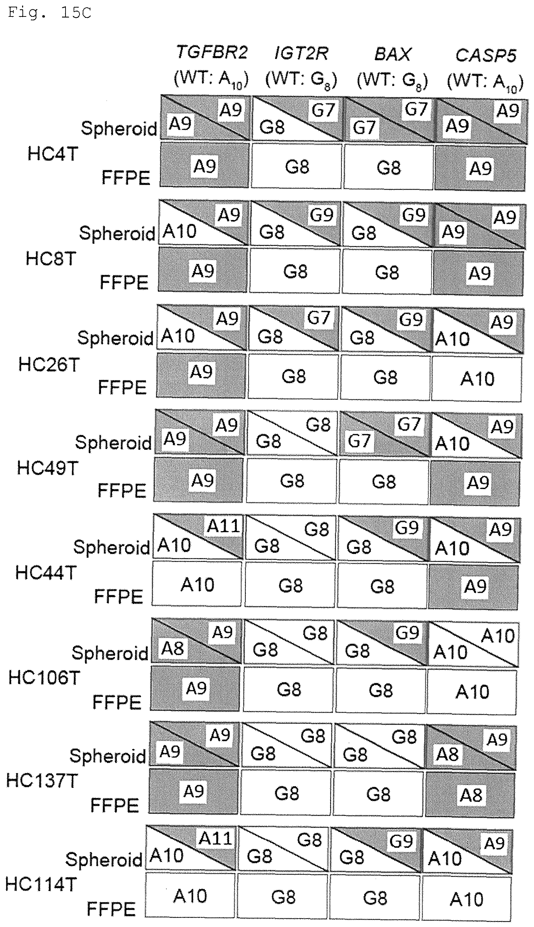

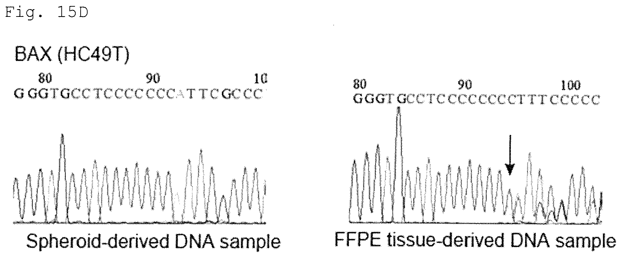

To test the quality of the DNA samples further, we performed sequencing of MMR deficiency target 4 genes using spheroid derived-DNA samples and FFPE tissue-derived DNA samples in MSI-H cases. The heterozygous mutations detected with spheroid derived-DNA samples were not confirmed with FFPE tissue-derived DNA samples (Fig. 15C). The frequency of frameshift mutations in the respective genes in spheroid-derived DNA samples were 75% for TGFBR2, 0% for IGFR2, 0% for BAX and 63% for CASP5. Mutation frequencies of all genes detected with FFPE tissue-derived DNA samples were lower than those with spheroid-derived DNA samples. It was likely because of normal cell DNA contamination. Fig. 15D is a representative sequence chromatograms. This case (HC49T) was interpreted as G7/G7 homozygous mutation of BAX in spheroid-derived DNA analysis (Fig. 15D, left). However, in FFPE tissue-derived DNA analysis, peaks representing wild type alleles from normal cells dominated, which prevented from determining if there was any mutation (Fig. 15D, right). Accordingly, spheroid derived-DNA samples allowed us more accurate MSI tests than FFPE tissue-derived DNA samples.

Example 2: Selection of colorectal cancer patients being sensitive to FGFR inhibitors

1. Methods

Chemicals

Erdafitinib (JNJ42756493, Active Biochem, pan-FGFR inhibitor), AZD4547 (Active Biochem, FGFR1-3 inhibitor), BGJ398 (AdooQ, FGFR1-3 inhibitor), BLU-554 (Selleck Chemicals, FGFR4 selective inhibitor), and rogoratinib (MedChemo Express, FGFR1-3 inhibitor) were dissolved and diluted in DMSO. Cetuximab (Erbitux, Merck) was dissolved in phosphate buffered saline (PBS).

Establishment of patient-derived spheroids

Spheroids used in this example were the patient-derived cancer spheroids established by culturing tumor-derived cells in the cancer medium containing 100 ng/ml bFGF (Peprotech) and 50 ng/ml EGF (Peprotech) and the spheroids established from normal mucosa cells in Example 1. The established cancer spheroid lines were cultured with the aforementioned cancer medium containing bFGF and EGF.

Preparation of luciferase-expressing spheroids

Luciferase-expressing spheroids were prepared as described in Example 1 by using a lentiviral vector.

Drug sensitivity tests in spheroids

Luciferase-expressing spheroids were cultured in 2-3 wells of a 12-well cell-culture plate (in 60-90 $\mu$l Matrigel). Spheroids were trypsinized, filtered through a 40-$\mu$m cell strainer (Corning), and diluted with the washing medium 4-8 times (based on the volume of Matrigel) to provide a concentration of $5 \times 10^4$ - $2 \times 10^6$ cells/ml depending on the growth rate and spheroid density. From the spheroid solution thus obtained, 300 $\mu$l was taken and the cells were resuspended in an equal volume of Matrigel. The spheroid cells in Matrigel were distributed to the wells of 96-well "white" cell-culture plates (Corning; 3 $\mu$l per well) on a plate heater at 37°C, and cultured overnight in 100 $\mu$l/well of the medium. Spheroids were rinsed with 100 $\mu$l/well of PBS, and incubated with 50 $\mu$l of 150 $\mu$g/ml luciferin in phenol red-free DMEM/F12 medium (Nacalai) for 10 min at room temperature (20-28°C). Luminescence was scored using a conventional imaging device (Gel Doc XR+, BioRad). After this initial measurement, spheroids in each well were rinsed with 100 $\mu$l of PBS, and cultured in 100 $\mu$l of the medium. Luminescence was determined on day 1 and day 3. Effects of a drug was determined by obtaining GEI (growth effect index), which is the growth rate of a group treated with the drug compared to a control group (a solvent-treated group). Results were shown using means in three independent experiments each conducted in triplicate.

Preparation of patient-derived spheroid xenograft (PDSX) models

Patient-derived spheroids were cultured in a 12-well culture plate to become confluent ($4 \times 10^5$ - $4 \times 10^6$ cells/plate) and the spheroids were rinsed twice with PBS and placed in a 1.5 ml tube with Matrigel. The volume of the spheroid suspension was adjusted to 100 $\mu$l with PBS and the suspension thus obtained was subcutaneously injected into 4-6 week female nude mice (BALB/c-nu) at $1 \times 10^5$ - $9 \times 10^5$ cells/mouse.

Drug sensitivity tests in PDSX models

PDSX mice were subjected to drug-dosing test when the mean estimated tumor volume reached 300 mm$^3$. Tumor volume (mm$^3$) was estimated by the following formula: length x width$^2$ /2. We excluded the following kinds of tumors from the tests as outliers; too small (< 100 mm$^3$), spontaneously shrinking, remaining unchanged in size, or deeply implanted and difficult to be measured.
Mice in each set were distributed into the control and treatment groups (n = 3-4 per group), with the latter included the followings; erdafitinib monotherapy, cetuximab monotherapy, and erdafitinb + cetuximab. The day of the dosing start was defined as day 0, with the tumor volume of each mouse measured twice a week for 3 weeks (days 0-21). For erdafitinib treatment, the mouse dosage was set as 10 mg/kg according to the previous report (Perera TPS et al. Mol Cancer Ther. 2017; 16(6): 1010-20), and mice were dosed orally by allowing them to take a gel (MediGel® Sucralose, ClearH2O) containing the drug at 10 mg/mL in DMSO ad libitum as water substitute. For cetuximab treatment, the mouse dosage was set according to the following formula: mouse dose (mg/kg) = human dose (mg/kg) $\times$ 37 (hKm) / 3 (mKm) where Km indicates human or mouse body surface coefficient, and mice were injected intraperitoneally (i.p.) at 250 $\mu$g per injection, twice a week for three weeks.
The relative tumor volume was estimated by calibration to the initial tumor volume on day 0. To evaluate the drug dosing effects, the ratio of tumor volume on day 21 to that on day 0 was compared among the four groups.

Histological examinations

Paraffin-embedded tissues of PDSXs were prepared according to the standard procedures. Sections were stained with H&E or immunostained for Ki67.

Preparation of DNA from spheroids

Spheroids in Matrigel were suspended in Cell Recovery Solution (Corning), and collected in 1.5 ml-tubes. Matrigel was digested at 4°C with rotation for 30-60 min. Spheroids were centrifuged at 200 $\times$ g for 5 min, and washed with PBS twice at 4°C. DNA was purified using DNeasy Blood & Tissue Kit (Qiagen).

Array-based comparative genomic hybridization (CGH) analysis

Array-based CGH analysis was performed with Agilent SurePrint G3 human CGH microarray 1x1M (G4447A, Agilent, Santa Clara, CA, USA) and Genomic DNA ULS Labeling Kit (#5190-0419, Agilent) according to the manufacturer's instructions. Human genomic DNAs (G1521 and G1471, Promega, Madison, WI, USA) were used as references and controls. The array slides were scanned using Agilent G4900DABA microarray scanner (Agilent).

Preparation of RNA from spheroids

Spheroids in Matrigel were suspended in Cell Lysis Solution (TaKaRa), and RNA was purified using NucleoSpin® RNA kit (#U0955C, TaKaRa).

Microarray analysis of RNA

Array-based mRNA expression analysis was performed with SurePrint G3 Human Gene Expression 8 x 60k version 3.0 (G4851C, Agilent) and Quick Amp Labeling Kit (#5190-0442, Agilent) according to the manufacturer's instructions. The array slides were scanned using Agilent G4900DA SureScan microarray scanner (Agilent).

Statistical analysis

Data were analyzed using one-way and two-way ANOVA followed by Tukey's post-test using GrafPad Prism ver.6 (GraphPad software. Inc.).

2. Results

(1) Suppression of *in vitro* growth of patient-derived colorectal cancer spheroids by fibroblast growth factor receptor inhibitors

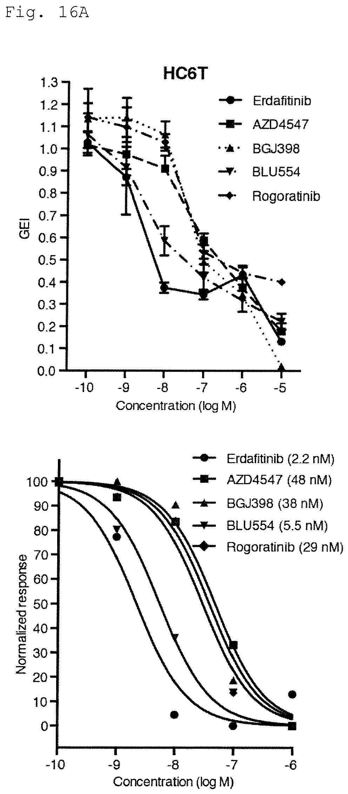

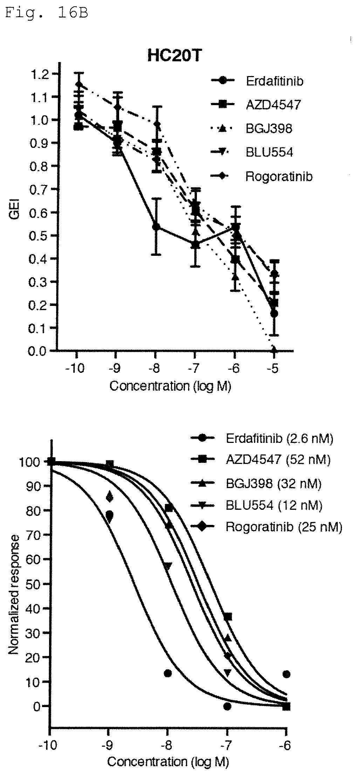

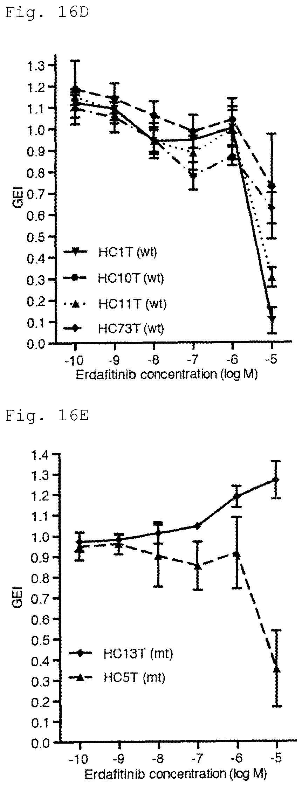

The spheroid lines shown in Table 3 were used in this example. We first treated three spheroid lines (HC6T, HC9T, and HC20T) with an FGFR inhibitor (erdafitinib, AZD4547, BGJ398, BLU554 or rogoratinib), and determined GEI. These spheroid lines (HC6T, HC9T and HC20T) contained no hot-spot mutations in any of *KRAS, NRAS* and *BRAF* genes (Table 3). Also, the growth of HC6T and HC9T was heavily dependent on bFGF (data not shown). As shown in Figs. 16A-16C, all the five FGFR inhibitors suppressed the growth of these spheroid lines in a dose-dependent manner. Among them, erdafitinib was most potent ($IC_{50}$: 2.2-3.1 nM).

Table 3

Characteristics of each spheroid used in this example

| Tumor ID | Patient | | | Tumor | | | Mutations in 50 cancer-related genes |
|---|---|---|---|---|---|---|---|
| | Age | Sex | Location | Histology | pStage | |
| HC1T | 81 | F | ascending | Well-Mod | 4 | PIK3CA |
| HC5T | 63 | M | transverse | Well-Mod | 4 | PIK3CA, KRAS (G12V), TP53 |
| HC6T | 57 | M | sigmoid | Well-Mod | 4 | TP53 |
| HC7T | 68 | F | sigmoid | Well-Mod | 2 | - |
| HC8T | 66 | F | transverse | Well-Mod | 1 | ERBB4, MLH1, PIK3CA, SRC |
| HC9T | 73 | M | ascending | Well-Mod | 2 | NOTCH1, TP53 |
| HC10T | 70 | F | cecum | Well-Mod | 2 | APC, MET, CDH1, TP53 |
| HC11T | 74 | M | transverse | Muc | 3 | TP53 |
| HC13T | 66 | M | sigmoid | Poor | 3 | BRAF (V600E), TP53 |
| HC20T | 66 | F | sigmoid | Well-Mod | 4 | ERBB4, MLH1, TP53, SMAD4 |
| HC21T | 52 | M | rectum | Well-Mod | 4 | TP53 |
| HC73T | 75 | M | sigmoid | Well-Mod | 4 | ERBB2, APC, TP53 |

Amino acid replacements are shown with amino acids before and after replacement at the affected codon.
Note that 50.5% of mutations in the APC gene was detectable in the test.
Well, well differentiated adenocarcinoma; Mod, moderately differentiated adenocarcinoma; Poor, poorly differentiated adenocarcinoma: Muc, mucinous carcinoma.

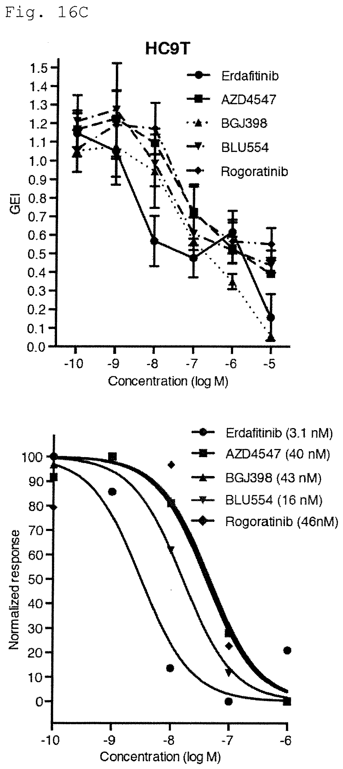

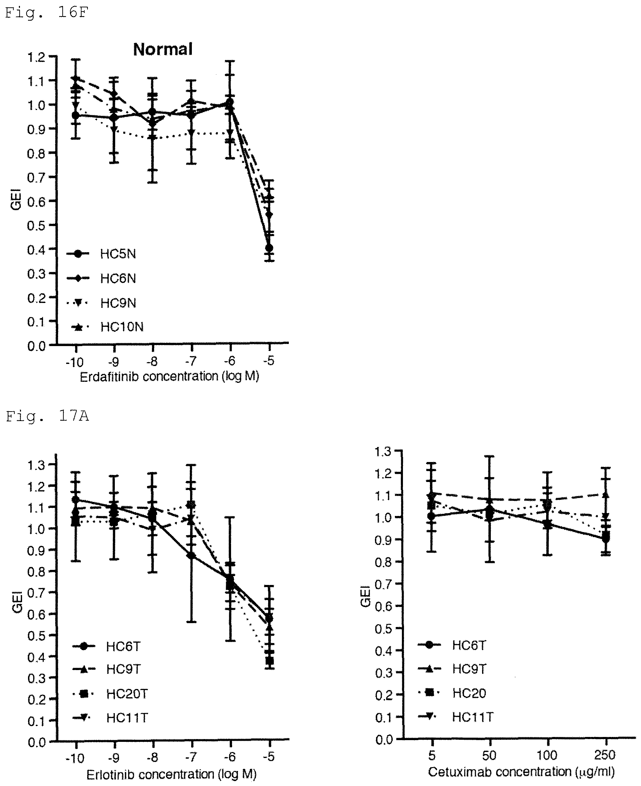

We then tested six spheroid lines that did not depend on bFGF for *in vitro* growth (HC1T, HC10T, HC11T, HC73T, HC5T and HC13T). Erdafitinib showed no growth inhibition on the six spheroid lines at clinically relevant doses (0.01-1 $\mu$M) (Figs. 16C and 16D) despite that HC1T, HC10T, HC11T, and HC73T did not have any mutations in *RAS/RAF* genes while HC5T and HC13T did. These results suggest that their growth was stimulated through other growth factor receptors such as epidermal growth factor receptor (EGFR), or through the activating mutation(s) in RAS/RAF proteins in HCT5T and HCT13T. Erdafitinib hardly suppressed the growth of normal colonic epithelial stem cell spheroids at concentrations below 1 $\mu$M (Fig. 16F), predicting minimal adverse effects on normal colonic mucosa associated with FGFR inhibition.

(2) Growth suppression of colorectal cancer spheroids by combination of FGFR and EGFR inhibitors

We tested effects of a combination of FGFR and EGFR inhibitors on the colorectal cancer spheroid growth. Among EGFR inhibitors, anti-EGFR antibodies cetuximab and panitumumab have already been approved for the treatment of *RAS/RAF* mutation-free colorectal cancer. However, cetuximab failed to suppress the *in vitro* growth of spheroids (Fig. 17A, right). It was likely because of its poor permeability through Matrigel. Accordingly, we employed erlotinib, one of small-molecule tyrosine kinase inhibitors, as an EGFR inhibitor, instead.

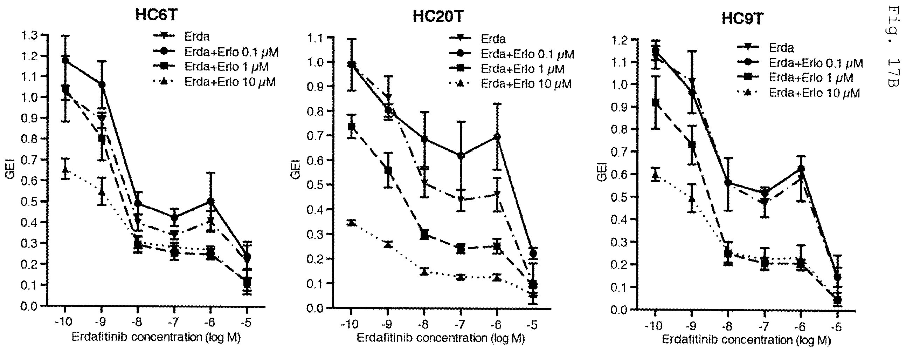

Erlotinib showed dose-dependent growth inhibition on *RAS*/*RAF* mutation-free spheroid lines (HC6T, HC9T, HC11T, and HC20T) (Fig. 17A, left). Erlotinib showed a slight growth-promoting effect on HC20T at 0.1 $\mu$M. We then tested treatment with a combination of erdafitinib and erlotinib on HC6T, HC9T and HC20T. Growth suppression was enhanced in the presence of both inhibitors compared with either monotherapy at a clinically relevant dose of 1 $\mu$M erlotinib and 0.01 $\mu$M erdafitinb (HC6T , $p < 0.05$; HC9T, $p < 0.01$; and HC20T, $p < 0.01$) (Fig. 17B). In HC20T, erlotinib at 0.1 $\mu$M rather enhanced its growth (not significant).

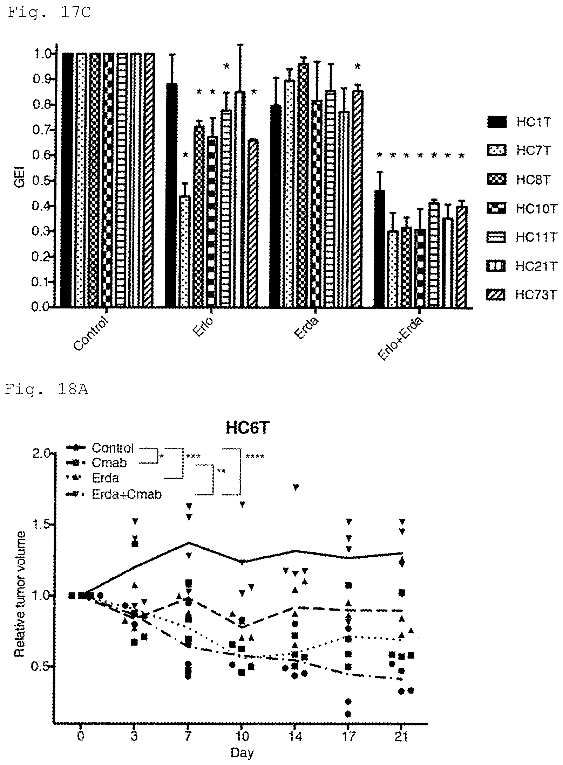

Furthermore, we screened for the effects of the combination of erdafitinib and erlotinib on other seven spheroid lines that were free of *RAS*/*RAF* hot-spot mutations including the four that were almost insensitive to erdafitinb alone (HC1T, HC10T, HC11T, and HC73T; Fig. 16D). All the seven lines showed 50% or less of GEI ($p < 0.05$-0.0001, Fig. 17C). These results show that double blockade of FGFR and EGFR can be more efficacious than either monotherapy for colorectal cancer.

(3) Reduction of PDSX tumor sizes with erdafitinib and/or cetuximab at clinically relevant doses

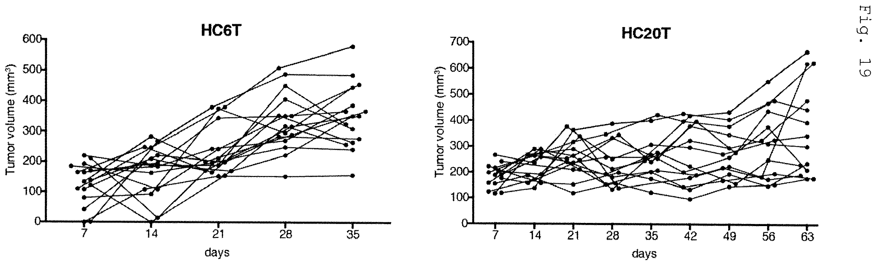

To determine the potential efficacy in blocking the FGFR and/or EGFR signaling in tumor-bearing mice, we established PDSX mice from two colorectal cancer spheroids lines, HC6T and HC20T, that responded well to both FGFR and EGFR inhibitors *in vitro* (Fig. 19). We divided PDSX mice into four treatment groups: control (solvent only), cetuximab monotherapy, erdafitinib monotherapy, and combination therapy of erdafitinib and cetuximab (erdafitinib + cetuximab). Each group was treated with erdafitinib and/or cetuximab (or solvent) at s clinically relevant dose for 21 days, and tumor volumes were estimated.

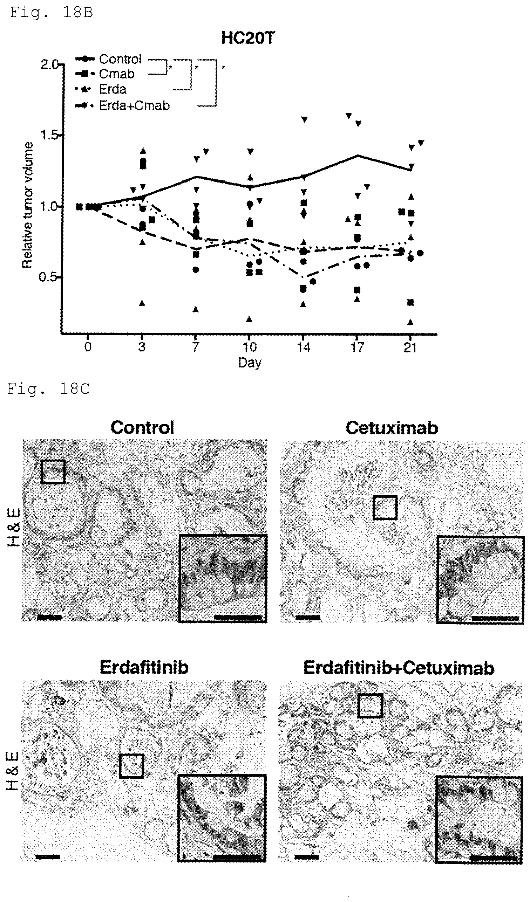

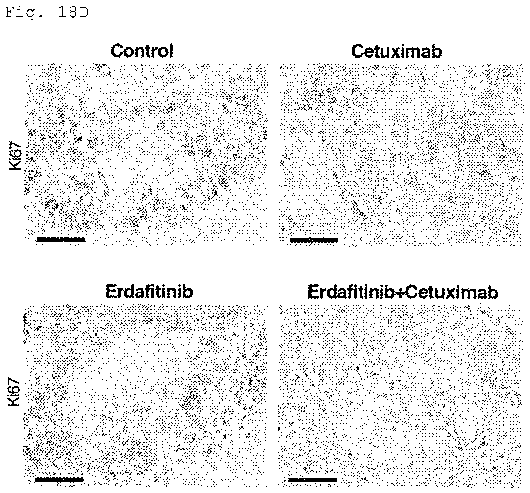

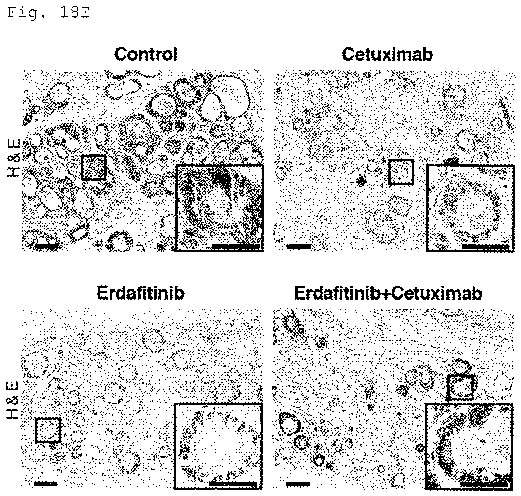

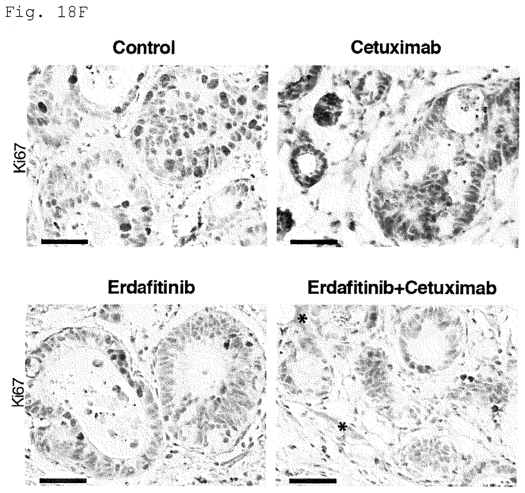

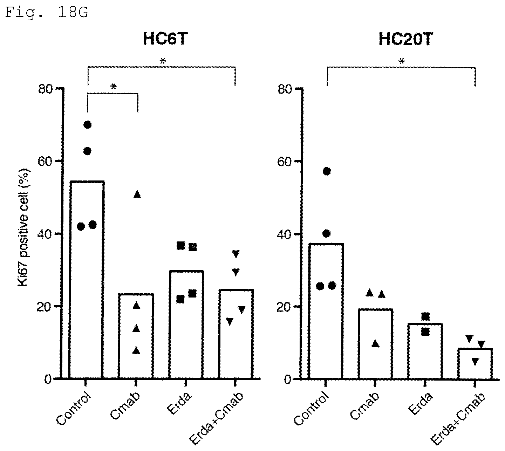

In HC6T-PDSX mice, cetuximab monotherapy, erdafitinib monotherapy and erdafitinib + cetuximab combination reduced the tumor volume with statistically significant differences from the control tumors ($p < 0.05$-0.0001; Fig. 18A). There was also a significant difference between cetuximab alone and erdafitinib + cetuximab ($p < 0.05$). In contrast, there was no statistically significant difference between erdafitinib alone and erdafitinib + cetuximab. In HC20T-PDSX, erdafitinib + cetuximab combination suppressed the tumor growth significantly compared with the control ($p < 0.05$), while cetuximab or erdafitinib alone also had similar tumor growth inhibition over the control ($p < 0.05$; Fig. 18B). The proportion of proliferating Ki-67-positive cells was reduced significantly in the combination therapy group, although no discernible changes were found in the histological features of PDSX tumors by the treatment (Figs. 18C-18G).

(4) FGF and FGFR gene copy numbers and mRNA expression levels in patient-derived colorectal cancer spheroids

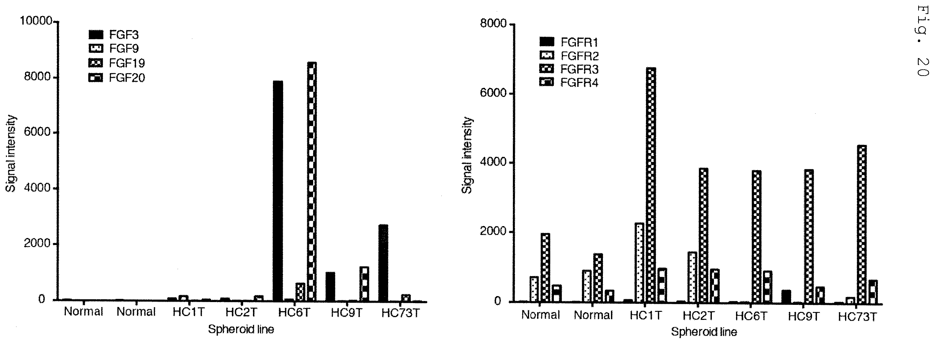

We examined whether there are changes in the copy numbers of the FGF9 and FGFR1-4 genes in patient-derived colorectal cancer spheroids. As shown in Table 4, no increase in the copy numbers of FGFR1 to 4 (normal value was 2) was observed in HC6T, HC9T, and HC20T, in which the FGFR inhibitor was effective alone. An increase in copy number was observed with HC11T, in which the FGFR inhibitor had no effect. As for FGF9, an increase in copy number was observed in HC6T and HC9T, in which the FGFR inhibitor was effective, and in HC11T and HC73T, in which the FGFR inhibitor was not effective. Further, mRNA expression levels of FGF3, 9, 19 and 20 and FGFR1 to 4 were examined, but no correlation with the response to the FGFR inhibitor was observed (Fig. 20). These results indicate that the sensitivity of colorectal cancer patients to FGFR inhibitors cannot be evaluated based on the copy number or mRNA expression level of FGF or FGFR gene.

Table 4

| genome | gene | HC1T | HC6T | HC9T | HC11T | HC20T | HC73T |
|---|---|---|---|---|---|---|---|
| 13 | FGF9 | 2.0 | 3.2 | 4.3 | 3.5 | 2.0 | 3.2 |
| 8 | FGFR1 | 2.0 | 1.4 | 1.5 | 2.5 | 2.0 | 1.5 |
| 10 | FGFR2 | 2.0 | 1.4 | 2.0 | 2.6 | 2.0 | 2.0 |
| 4 | FGFR3 | 2.0 | 1.4 | 1.2 | 2.0 | 1.5 | 2.0 |
| 5 | FGFR4 | 2.0 | 2.0 | 1.5 | 2.0 | 1.5 | 1.5 |
| sensitivity | | R | S | S | R | S | R |
| R: resistant, S: sensitive. | | | | | | | |

**Claims**

1.  A method of preparing a cancer spheroid, comprising
    culturing a cell population obtained from a cancer tissue of a patient in a medium containing
    a ROCK inhibitor, a TGF-β type I receptor inhibitor, EGF and bFGF; and
    an agent selected from an adenosine receptor agonist, a PPAR agonist, a calcium-activated potassium-channel (KCa) activator, an NMDA-type glutamate receptor agonist, a histamine H3 receptor agonist, and a retinoic acid receptor (RXR) β2 agonist.

2.  The method of claim 1, wherein the cancer is colorectal cancer.

3.  The method of claim 1 or 2, wherein the agent is an adenosine receptor agonist.

4.  The method of claim 3, wherein the adenosine receptor agonist is NECA or CV1808.

5.  The method of any one of claims 1-4, wherein the agent is selected from NECA, CV1808, GW0742, GW7647, S26948, L-165,041, DCEBIO, SKA-31, NMDA, imepip and AC55649.

6.  The method of any one of claims 1-5, wherein the ROCK inhibitor is Y27632.

7.  The method of any one of claims 1-6, wherein the TGF-β type I receptor inhibitor is SB431542.

8.  The method of any one of claims 1-7, wherein the medium further comprises serum.

9.  A composition comprising a cancer spheroid obtained by the method of any one of claims 1-8 or a cancer spheroid derived therefrom.

10. A method of evaluating drug sensitivity of a patient, comprising
    preparing a cancer spheroid by the method of any one of claims 1-8, and
    contacting the cancer spheroid thus obtained or a cancer spheroid derived therefrom with a candidate agent *in vitro*.

11. A method of screening a therapeutic agent for a cancer, comprising
    preparing a cancer spheroid by the method of any one of claims 1-8, and
    contacting the cancer spheroid thus obtained or a cancer spheroid derived therefrom with a candidate agent *in vitro*.

12. A method of preparing a patient-derived spheroid xenograft (PDSX) model, comprising
    preparing a cancer spheroid by the method of any one of claims 1-8, and
    transplanting the cancer spheroid thus obtained or a cancer spheroid derived therefrom to a non-human animal.

13. A method of evaluating drug sensitivity of a patient, comprising
    preparing a PDSX model by the method of claim 12, and administering a candidate agent to the PDSX model thus obtained.

14. A method of screening a therapeutic agent for a cancer, comprising
    preparing a PDSX model by the method of claim 12, and administering a candidate agent to the PDSX model thus obtained.

15. A method of detecting microsatellite instability (MSI), comprising
    preparing a cancer spheroid by the method of any one of claims 1-8, and
    determining MSI status or a mutation of a target gene of a mismatch repair gene in the cancer spheroid thus obtained or a cancer spheroid derived therefrom.

16. A method of selecting a colorectal cancer patient being sensitive to an FGFR inhibitor, comprising treating a cancer spheroid or a patient-derived spheroid xenograft (PDSX) model derived from a colorectal cancer patient with an FGFR inhibitor, and
    determining response to the FGFR inhibitor of the cancer spheroid or the PDSX model, and
    selecting the colorectal cancer patient when the response to the FGFR inhibitor is positive.

**17.** The method of claim 16, wherein the treating comprises treating a cancer spheroid derived from a colorectal cancer patient with an FGFR inhibitor.

**18.** The method of claim 17, wherein the response to the FGFR inhibitor is determined based on growth or a histological feature of the cancer spheroid.

**19.** The method of claim 16, wherein the treating comprises treating a PDSX model with an FGFR inhibitor.

**20.** The method of claim 19, wherein the response to the FGFR inhibitor is determined based on the volume or a histological feature of a tumor tissue of the PDSX model.

**21.** The method of any one of claims 16-20, wherein the method further comprises preparing the cancer spheroid from a cell population obtained from a cancer tissue of the colorectal cancer patient.

**22.** The method of any one of claims 16-21, wherein the method further comprises preparing the PDXS model by transplanting a cancer spheroid derived from the colorectal cancer patient to a non-human animal.

**23.** The method of any one of claims 16-22, wherein the FGFR inhibitor is selected from erdafitinib, rogoratinib, AZD4547, BGJ398, BLU554, LY2874455, ASP5878, and ARQ087.

**24.** The method of any one of claims 16-23, wherein the FGFR inhibitor is erdafitinib.

**25.** A pharmaceutical composition for treating colorectal cancer comprising an FGFR inhibitor.

**26.** The pharmaceutical composition of claim 25, wherein the pharmaceutical composition is administered to a colorectal cancer patient selected by the method of any one of claims 16-54.

**27.** The pharmaceutical composition of claims 25 or 26, wherein the FGFR inhibitor is selected from erdafitinib, rogoratinib, AZD4547, BGJ398, BLU554, LY2874455, ASP5878, and ARQ087.

**28.** The pharmaceutical composition of any one of claims 25-27, wherein the FGFR inhibitor is erdafitinib.

**29.** The pharmaceutical composition of any one of claims 25-28, wherein the pharmaceutical composition is used in combination with an EGFR inhibitor.

**30.** The pharmaceutical composition of claim 29, wherein the EGFR inhibitor is cetuximab.

Fig. 1

Primary cancer      Spheroid

HC1T (Low-grade, w/ goblet cell)

HC16T (Low-grade, w/o goblet cell)

HC8T (High-grade, w/o goblet cell)

Fig. 2A

Fig. 2B

Fig. 2C

Fig. 3A

Fig. 3B

Fig. 3C

Fig. 3D

Fig. 4A

Fig. 4B

Fig. 4C

HC52T

Fig. 4D

Fig. 5

Fig. 6A

Fig. 6B

Fig. 6C

Fig. 7A

Fig. 7B

Fig. 8A

Fig. 8B

1 Isoproterenol hydrochloride
2 L-Cysteinesulfinic acid
3 L-165,041
4 THIP hydrochloride
5 WAY 200070
6 5-Iodo-A-85380 dihydrochloride
7 Eletriptan hydrobromide
8 Dimaprit dihydrochloride
9 GW 7647
10 Procaterol hydrochloride
11 GW 3965 hydrochloride
12 WAY 161503 hydrochloride
13 A 61603 hydrobromide
14 SR 11237
15 BW 723C86 hydrochloride
16 α-Methyl-5-hydroxytryptamine maleate
17 CITCO
18 AM580
19 AC 55649
20 PNU 282987

21 Pindolol
22 Clofibric acid
23 5-Methylfurmethiodide
24 Cisplatin
25 Spaglumic acid
26 m-Chlorophenylbiguanide hydrochloride
27 Buspirone hydrochloride
28 ATPA
29 RWJ 21757
30 Histamine dihydrochloride
31 CV 1808
32 (R)-(-)-Apomorphine hydrochloride
33 (±)-Bay K 8644
34 Dobutamine hydrochloride
35 Melatonin
36 N α-Methylhistamine dihydrochloride
37 DCEBIO
38 Oleamide
39 FPL 64176
40 Spermidine trihydrochloride

41 SKA 31
42 Cisapride
43 Cirazoline hydrochloride
44 Rilmenidine hemifuramate
45 Muscimol
46 Quinolinic acid
47 (-)-U-50488 hydrochloride
48 1-EBIO
49 (RS)-AMPA hydrobromide
50 TTNPB
51 WY 14643
52 T 0901317
53 Ibotenic acid
54 UK 14,304 tartrate
55 SKF 77434 hydrobromide
56 PSN 375963 hydrochloride
57 5-Carboxamidotryptamine maleate
58 m-3M3FBS
59 CD 437
60 CP 93129 dihydrochloride

61 (-)-Quinpirole hydrochloride
62 Formoterol hemifumarate
63 S26948
64 PRE-084 hydrochloride
65 NMDA
66 Lobeline hydrochloride
67 Pilocarpine hydrochloride
68 (E)-Capsaicin
69 BRL 37344, sodium salt
70 ZD 7114 hydrochloride
71 ABT 724 trihydrochloride
72 L-Quisqualic acid
73 NECA
74 Immepip dihydrobromide
75 Amthamine dihydrobromide
76 GW 0742
77 Guanabenz acetate
78 GW 405833
79 Ceramide
80 Olvanil

Fig. 9A

Fig. 9B

Fig. 9C

Fig. 9D

Fig. 10A

Fig. 10B

Fig. 11A

**HC13T PDSX**

Fig. 11B

**HC13T PDSX**

95% Confidence Intervals (Sidak)

Fig. 11C

**HC13T PDX**

Fig. 11D

**HC13T PDX**

95% Confidence Intervals (Sidak)

Fig. 12A

Fig. 12B

Fig. 12C

Fig. 12D

Fig. 12E

Fig. 12F

Fig. 12G

### Individual tumors (Day 22)

Fig. 13A

Fig. 13B

Fig. 13C

Fig. 13D

## Individual tumors (Day 22)

Fig. 14A

Fig. 14B

Fig. 14C

Fig. 14D

Fig. 14E

**TGFBR2**

$A_{10}/A_{10}$ (Wild type)

HC33T

$A_9/A_9$ (Homozygous mutation)

HC4T

$A_{10}/A_9$ (Heterozygous mutation)

HC26T

**BAX**

$G_8/G_8$ (Wild type)

HC61T

$G_7/G_7$ (Homozygous mutation)

HC4T

$G_8/G_9$ (Heterozygous mutation)

HC26T

54

Fig. 15A

Fig. 15B

Fig. 15C

Fig. 15D

BAX (HC49T)

Spheroid-derived DNA sample

FFPE tissue-derived DNA sample

Fig. 16A

Fig. 16B

Fig. 16C

Fig. 16D

Fig. 16E

Fig. 16F

Fig. 17A

Fig. 17C

Fig. 18A

Fig. 18B

Fig. 18C

Fig. 18D

Fig. 18E

**Control**

**Cetuximab**

**Erdafitinib**

**Erdafitinib+Cetuximab**

Fig. 18F

Fig. 18G

Fig. 19

Fig. 20

## INTERNATIONAL SEARCH REPORT

| International application No. |
|---|
| PCT/JP2018/044894 |

### A. CLASSIFICATION OF SUBJECT MATTER

Int.Cl.  C12N5/09(2010.01)i, A61K45/00(2006.01)i, A61P35/00(2006.01)i,
          C12Q1/02(2006.01)i, C12Q1/6886(2018.01)i, G01N33/15(2006.01)i,
          G01N33/50(2006.01)i

According to International Patent Classification (IPC) or to both national classification and IPC

### B. FIELDS SEARCHED

Minimum documentation searched (classification system followed by classification symbols)

Int.Cl.  C12N5/09, A61K45/00, A61P35/00, C12Q1/02, C12Q1/6886, G01N33/15,
          G01N33/50

| Documentation searched other than minimum documentation to the extent that such documents are included in the fields searched | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)
CAplus/REGISTRY/MEDLINE/EMBASE/BIOSIS /WPIDS (STN)

### C. DOCUMENTS CONSIDERED TO BE RELEVANT

| Category* | Citation of document, with indication, where appropriate, of the relevant passages | Relevant to claim No. |
|---|---|---|
| A | JP 2014-516562 A (KONINKLIJKE NEDERLANDSE AKADEMIE VAN WETENSCHAPPEN) 17 July 2014, claims 22, 31, 34, example 5 & US 2014/0243227 A1 (claims 22, 31, 34, example 5) & WO 2012/168930 A2 & EP 2718422 A2 & AU 2012265814 A & CA 2838492 A & KR 10-2014-0037210 A & CN 104024401 A & RU 2013158140 A & NZ 619314 A & ES 2650980 T | 1-15 |
| A | JP 2008-022743 A (SCIVAX CORPORATION) 07 February 2008, claims (Family: none) | 1-15 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 25 February 2019 (25.02.2019) | 05 March 2019 (05.03.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

International application No.

PCT/JP2018/044894

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2017-205021 A (JSR CORPORATION) 24 November 2017, claims & WO 2016/047801 A1 | 1-15 |
| A | RZESKI, Wojciech et al., "Glutamate antagonists limit tumor growth.", Biochemical pharmacology, 15 October 2002, vol. 64, no. 8, pp. 1195-1200, (abstract) | 1-15 |
| A | OHATA, Hirokazu et al., "Induction of the Stem-like Cell Regulator CD44 by Rho Kinase Inhibition Contributes to the Maintenance of Colon Cancer-Initiating Cells", Cancer Research, 2012, vol. 72, no. 19, pp. 5101-5110, (OF2-3, fig. 2) | 1-15 |
| Y | PAULI, Chantal et al., "Personalized In Vitro and In Vivo Cancer Models to Guide Precision Medicine.", Cancer Discov, May 2017, vol. 7, no. 5, pp. 462-477, (fig. 2, 3, 5-7) | 16-24 |
| Y | VAN, de Wetering M. et al., "Prospective derivation of a living organoid biobank of colorectal cancer patients.", Cell, 07 May 2015, vol. 161, no. 4, pp. 933-945, (fig. 1, 6) | 16-24 |
| Y | JP 2013-516437 A (BIORÉALITÉS) 13 May 2013, example 3 & US 2011/0177063 A1 & US 2011/0177062 A1 & WO 2011/083088 A2 (example 3) & EP 2542585 A2 & CA 2786417 A & SG 182335 A & CN 102947337 A & KR 10-2013-0028049 A & NZ 601584 A & ZA 201205003 B & ZA 201205004 B | 16-24 |
| X/Y | WO 2016/091849 A1 (BAYER PHARMA AKTIENGESELLSCHAFT) 16 June 2016, claims 1, 14 & JP 2017-538708 A & US 2018/0333418 A & EP 3229800 A2 & TW 201628655 A & AU 2015359538 A & CA 2970181 A & CN 106999592 A & KR 10-2017-0090431 A & MX 2017007656 A & EA 201791236 A & CL 2017001487 A & IL 252187 D & BR 112017012287 A & SG 11201704090W A | 25-28/16-24 |
| X/Y | WO 2017/100642 A1 (REGENERON PHARMACEUTICALS, INC.) 15 June 2017, claims & EP 3387017 A1 & CA 3007644 A & AU 2016366521 A & CN 108602890 A & JP 2018-536682 A | 25-28/16-24 |
| X/Y | VERSTRAETE, Maud et al., "In vitro and in vivo evaluation of the radiosensitizing effect of a selective FGFR inhibitor (JNJ-42756493) for rectal cancer", BMC Cancer, 2015, 15, 946, (abstract) | 25-28/16-24 |

Form PCT/ISA/210 (continuation of second sheet) (January 2015)

## INTERNATIONAL SEARCH REPORT

| International application No. |
| --- |
| PCT/JP2018/044894 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X/Y | YAO, Tingjing et al., "AZD-4547 exerts potent cytostatic and cytotoxic activities against fibroblast growth factor receptor (FGFR)-expressing colorectal cancer cells", Tumor Biology, 2015, vol. 36, no. 7, pp. 5641-5648, (abstract) | 25-28/16-24 |
| X/Y | TURKINGTON, R. C. et al., "Fibroblast growth factor receptor 4 (FGFR4):a targetable regulator of drug resistance in colorectal cancer", Cell Death & Disease, 2014, vol. 5, no. 2, e1046, (abstract) | 25-28/16-24 |
| X/Y | US 2014/0308284 A1 (KIM, Bo-Gyou) 16 October 2014, abstract, example 3 & EP 2786765 A2 & KR 10-2014-0119623 A | 25-28/16-24 |
| X/Y | JP 2017-526671 A (DAIICHI SANKYO EUROPE GMBH) 14 September 2017, example 23 & US 2017/0226213 A (example 23) & WO 2016/023894 A & EP 3180356 A1 & TW 201619198 A | 25-28/16-24 |

Form PCT/ISA/210 (continuation of second sheet) (January 2015)

## INTERNATIONAL SEARCH REPORT

International application No.

PCT/JP2018/044894

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:
  See extra sheet

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

| International application No. |
| --- |
| PCT/JP2018/044894 |

<Continuation of Box No. III>

Claims 1-8 have the special technical feature of "a method for preparing cancer spheroids, the method including culturing a cell population from cancer tissue of a patient in a medium containing an agent selected from among a ROCK inhibitor, … and a retinoic acid receptor (RXR) β2 agonist", and thus are classified as invention 1.

Claims 9-15 are dependent on claims 1-8, and classified as invention 1.

Claims 16-24 share the common technical feature of "cancer spheroids" with claim 1 classified as invention 1. However, this technical feature does not make a contribution over the prior art in light of the description in paragraphs [00760] and [0007], and thus cannot be considered a special technical feature. Also, there are no other identical or corresponding special technical features between claims 16-24 and claim 1.

In addition, claims 16-24 are not dependent on claim 1. Further, claims 16-24 are not substantially identical or equivalent to any of the claims classified as invention 1.

Therefore, claims 16-24 cannot be classified as invention 1.

In addition, claims 16-24 have the special technical feature of "a method for selecting a patient with colorectal cancer sensitive to FGFR inhibitor, the method comprising: treating cancer-spheroids derived from a patient with colorectal cancer or a patient-derived spheroid xenograft (PDSX) model using an FGFR inhibitor, … investigating the response, … selecting a patient with colorectal cancer", and thus are classified as invention 2.

Claims 25-30 share the common technical feature of "cancer" with claims 1-15 classified as invention 1. However, this technical feature does not make a contribution over the prior art in light of common technical knowledge, and thus cannot be considered a special technical feature. Also, there are no other identical or corresponding special technical features between claims 25-30 and claim 1.

Furthermore, claims 25-30 are not dependent on claim 1. Moreover, claims 25-30 are not substantially identical or equivalent to any of the claims classified as invention 1.

Thus, claims 25-30 cannot be classified as invention 1.

In addition, it is recognized that claims 25-30 are substantially identical or equivalent to claims 16-24 classified as invention 2.

Therefore, claims 25-30 are classified as invention 2.

As explained above, claims 1-15 are classified as invention 1, and claims 16-30 are classified as invention 2.

Form PCT/ISA/210 (extra sheet) (January 2015)

**INTERNATIONAL SEARCH REPORT**

International application No.

PCT/JP2018/044894

\<Regarding the subject of the examination\>
    Claims 1-15 relate to a method including culturing in a medium containing compounds defined by the desired properties of "a ROCK inhibitor", "a TGF-β type I receptor inhibitor", "an adenosine receptor agonist", "a PPAR agonist", "a calcium activated potassium channel (Kca) activator", "an NMDA type glutamic acid receptor agonist", "a histamine H3 receptor agonist", and "a retinoic acid receptor (RXR) β2 agonist".

    In addition, "a ROCK inhibitor", "a TGF-β type I receptor inhibitor", "an adenosine receptor agonist", "a PPAR agonist", "a calcium activated potassium channel (Kca) activator", "an NMDA type glutamic acid receptor agonist", "a histamine H3 receptor agonist", and "a retinoic acid receptor (RXR) β2 agonist" include all compounds having such properties, and thus it is recognized that what is disclosed within the meaning of PCT Article 5 is merely an extremely small portion of the claimed compounds, and thus it is recognized that the claims lack support from the disclosure of the description within the meaning of PCT Article 6.

    Furthermore, with regard to the "a ROCK inhibitor", "a TGF-β type I receptor inhibitor", "an adenosine receptor agonist", "a PPAR agonist", "a calcium activated potassium channel (Kca) activator", "an NMDA type glutamic acid receptor agonist", "a histamine H3 receptor agonist", and "a retinoic acid receptor (RXR) β2 agonist", the scope of compounds having such properties cannot be specified even in consideration of the common technical knowledge at the date of filing. Thus, claims 1-15 do not satisfy the requirement of clarity stipulated in PCT Article 6.

    Therefore, the search was carried out for the method including culturing in a medium containing compounds specifically described in the description and specified in claims 4-7.

Form PCT/ISA/210 (extra sheet) (January 2015)

## REFERENCES CITED IN THE DESCRIPTION

**Patent documents cited in the description**

- JP 2017236374 A
- JP 2018195647 A

**Non-patent literature cited in the description**

- **KONDO J et al.** *Proc Natl Acad Sci U S A.,* 2011, vol. 108, 6235-40
- **VAN DE WETERING M et al.** *Cell,* 2015, vol. 161, 933-45
- **FUJII M et al.** *Cell Stem Cell,* 2016, vol. 18, 827-38
- **PAULI C et al.** *Cancer Discov.,* 2017, vol. 7, 462-77
- **MIYOSHI H et al.** *Science,* 2012, vol. 338, 108-13
- **MIYOSHI H ; STAPPENBECK TS.** *Nat Protoc.,* 2013, vol. 8, 2471-82
- **VANDUSSEN KL et al.** *Gut.,* 2015, vol. 64, 911-20
- Arch Pathol Lab Med. College of American Pathologists Consensus Statement 1999, 2000, vol. 124, 979-94
- *Gene,* 1991, vol. 108, 193-9
- **PERERA TPS et al.** *Mol Cancer Ther.,* 2017, vol. 16 (6), 1010-20